# EUROPEAN PATENT SPECIFICATION

(11) **EP 2 828 248 B1**
(45) Date of publication and mention of the grant of the patent: **23.01.2019**
(21) Application number: 13713752.7
(22) Date of filing: 22.03.2013
(51) Int. Cl.: C07D 311/30, C07D 493/04, A61K 31/352, A61P 31/14, A61P 31/20, C07D 405/04

(54) **FLAVONE DERIVATIVES AND THEIR USE**
FLAVON DERIVATE UND IHRE VERWENDUNG
DÉRIVÉS DE LA FLAVONE ET LEUR UTILISATION

(30) Priority: 23.03.2012 EP 12002056
(43) Date of publication of application: 28.01.2015
(73) Proprietor: Twincore GmbH, 30625 Hannover (DE); CNRS Centre National de la Recherche Scientifique, 75794 Paris Cedex 16 (FR); Université de Strasbourg, 67081 Strasbourg Cedex (FR)
(72) Inventor: PIETSCHMANN, Thomas, 30625 Hannover (DE); HAID, Sibylle, 30625 Hannover (DE); GENTZSCH, Juliane, 30625 Hannover (DE); GRETHE, Christina, 30625 Hannover (DE); DAVIOUD-CHARVET, Elisabeth, F-75794 Paris Cedex 16 (FR); LANFRANCHI, Don Antoine, F-20167 Sarrola Carcopino (FR); ELHABIRI, Mourad, F-75794 Paris Cedex 16 (FR); BENLLOCH-MARTIN, Xavier, F-67081 Strasbourg Cedex (FR)
(74) Representative: Lavoix
(86) International application number: PCT/EP2013/000890
(87) International publication number: WO 2013/139487

(56) References cited:
- WO-A1-2004/037237
- WO-A1-2007/069823
- WO-A2-01/03681
- WO-A2-2009/026166
- A. T. GUPTA ET. AL.: "Integrated ligand and structure based studies of flavonoids as fatty acid biosynthesis inhibitors of Plasmodium falciparum.", BIOORGANIC AND MEDICINAL CHEMISTRY LETTERS, vol. 20, 20 June 2010 (2010-06-20), pages 4779-4781, XP002681130, ISSN: 0960-894X, DOI: 10.1016/j.bcml.2010.06.120
- A. KARIOTI ET. AL.: "Identification of tyrosine inhibitors from Marrubium velutinum and Marrubium cylleneum.", BIOORGANIC AND MEDICINAL CHEMISTRY, vol. 15, 23 January 2007 (2007-01-23), pages 2708-2714, XP002681131,
- Y. KIKUCHI ET. AL.: "Studies on Nepalese Crude Drugs. XIV. New Flavonoids form the Root of Scutellaria prostata Jacq. ex Benth. .", CHEMICAL AND PHARMACEUTICAL BULLETIN, vol. 39, no. 6, 1 June 1991 (1991-06-01), pages 1466-1472, XP002681132,
- H. IWAMOTO ET. AL.: "Photoinduced Intramolecular Cyclization Reaction of 3-Substituted 2-Alkenoyl-1,4-benzoquinones.", BULLETIN OF THE CHEMICAL SOCIETY OF JAPAN, vol. 62, 1 November 1989 (1989-11-01), pages 3479-3487, XP002681133,
- D. O. LAUX ET. AL.: "Bausplendin, a dimethylenexioxyflavone from Bauhinia splendens.", PHYTOCHEMISTRY, vol. 24, no. 5, 1 May 1985 (1985-05-01), pages 1081-1084, XP002681134, ISSN: 0031-9422
- S. HAID ET. AL.: "A Plant-Derived Flavoloid Inhibits Entry of All HCV Genotypes Into Huma Hepatocytes.", GASTROENTEROLOGY, vol. 143, no. 1, 1 July 2012 (2012-07-01), pages 213-222,e1-e5, XP002681135, ISSN: 0016-5085, DOI: 10.1053/j.gastro.2012.03.036

## Description

### Field of the Invention

The present invention relates to flavone derivatives and to compositions containing one or more of these flavone derivatives. The present invention further relates to flavone derivatives or compositions for use in the treatment and/or prevention of a viral infection.

### Background of the Invention

Various flavonoids, methods to obtain them and their uses, e.g. to treat infectious conditions such as viral or parasite infections, are known in the art and have been widely reported in the literature. For instance, WO 01/03681 discloses the use of flavonoid compounds, such as the flavones cirsiliol and naringin, or coumarins in the treatment of viral or parasitic infections.

Gupta et al, Bioorganic & Medical Chemistry Letters June 20, 2010, 4779-4781 discloses flavonoids as inhibitors of *Plasmodium falciparum.* However Gupta et al., does not suggest the use of the said flavonoids as antiviral compounds.

Viruses are infectious agents that are found in virtually all life forms, including humans, animals, plants, fungi, and bacteria. Viruses often damage or kill the cells that they infect, causing disease in infected organisms. The difficulty in treating virus infections stems from the large number of variant viruses that can cause the same disease, as well as the inability of many drugs to disable a virus without disabling healthy cells.

Interestingly, the entry mechanisms are shared or reasonably conserved between enveloped virus particles, such as flaviviruses (e.g. hepatitis C virus (HCV), dengue virus, west nile virus), human immunodeficiency virus (HIV), herpesviruses, vesicular stomatitis virus (VSV), or influenza viruses. That is, virus-cell fusion is mediated by viral fusion proteins operating through shared mechanisms among enveloped viruses and modulating membrane properties of host and viral membranes.

### Flaviviruses

The *Flaviviridae* (also referred to as flaviviruses) are a family of viruses that infect a wide range of vertebrates and are spread through arthropods, mainly ticks and mosquitoes, or are transmitted parenterally (through blood) as well as sexually and vertically (from mother to child). The family Flaviviridae comprises three genera: Genus Flavi, Genus Hepaci, and Genus Pesti.

The genus *Flavi,* for example, includes the species Dengue Virus 1-4; West Nile Virus; Yellow Fever Virus; Tick-borne Encephalitis Virus; Japanese Encephalitis Virus; St. Louis Encephalitis Virus; Murray Valley Encephalitis Virus; Kunjin Encephalitis Virus; Rocio Encephalitis Virus; Russian Spring Summer Encephalitis Virus; Negeishi Virus; Kyasanur Forest Virus; Omsk Hemorrhagic Fever Virus; Powassan Virus; Louping Ill Virus; Rio Bravo Virus 1-7; Tyuleniy Virus; Ntaya Virus; Uganda S Virus/Zika Virus; and Modoc Virus.

The genus *Hepaci* includes the species Hepatitis C Virus and Hepatitis G Virus.

The genus *Pesti* contains viruses infecting non-human mammals, e. g. Bovine virus diarrhea 1-3.

The Flaviviruses are small (about 40 - 60 nm in diameter), enveloped, single-stranded RNA viruses with linear non-segmented genomes. The Flaviviridae genome is infectious and on average about 9.6 to 12.3 kilobase in length - it encodes around 10 genes. Viruses in this family are considered positive (+) sense because proteins are made directly from the template strand of RNA which is present in the viral capsid.

Major diseases caused by the *Flaviviridae* family include: Hepatitis C Virus Infection; Dengue fever; encephalitis; and hemorrhagic fever.

An overview on flaviviruses can, for example, be found in Westaway EG, et al.,"Flaviviridae", Intervirology 1985; 24(4): 183-92; Chambers TJ and Rice CM, "Molecular biology of the flaviviruses", Microbiol Sci. 1987; 4(7): 219-23; and Knipe DM and Peter MH, eds. Fields Virology; 5th ed. Vol. 2; Philidelphia, PA: Wolters Kluwer Health, 2007; p. 1102-1291.

### Human immunodeficiency virus (HIV)

HIV is a retrovirus. Two types of HIV have been characterized: HIV-1 and HIV-2, which share a common structure. HIV is roughly spherical with a diameter of about 120 nm. It is composed of two copies of positive single-stranded RNA that codes for the virus's nine genes enclosed by a conical capsid composed of 2,000 copies of the viral protein p24, which is surrounded by a matrix composed of the viral protein p17 thereby ensuring the integrity of the virion particle and the viral envelope. The viral envelope contains a glycoprotein complex, which enables the virus to attach to and fuse with target cells to initiate the infectious cycle. That is, HIV infects macrophages, CD4⁺ T cells, and dendritic cells of the host by the adsorption of glycoproteins on its surface to receptors on the target cell followed by fusion of the viral envelope with the cell membrane and the release of the HIV capsid into the cell.

### Herpesviruses

The *Herpesviridae* (also referred to as herpesviruses) are a large family of DNA viruses that cause diseases in animals, including humans. *Herpesviridae* can cause infections, such as oral and/or genital herpes, chickenpox and shingles, infectious mononucleosis, Burkitt's lymphoma, CNS lymphoma, retinitis, sixth disease etc. Herpesviruses all share a common structure. The virus particle is composed of relatively large double-stranded, linear DNA genomes encoding 100-200 genes encased within a protein cage (called capsid), which is itself wrapped in a protein layer containing both viral proteins and viral mRNAs and a lipid bilayer membrane called the envelope. All herpesviruses are nuclear-replicating, i.e. the viral DNA is transcribed to RNA within the infected cell's nucleus. Infection is initiated when a viral particle contacts a cell with specific types of receptor molecules on the cell surface. Following binding of viral envelope glycoproteins to cell membrane receptors, the viral particle is internalized and dismantled, allowing viral DNA to migrate to the cell nucleus.

### Vesicular Stomatitis Virus (VSV)

VSV is a member of the family Rhabdoviridae, genus Vesiculovirus. It is a small, compact virus, with only 11-12 kilobases in its genome. The virus carries as its genome a single-stranded, minus sense RNA. In order for the virus to replicate, it must use its own polymerase to create mRNAs and then translate those into proteins. Infected hosts usually show flu-like symptoms, e.g. fever.

### Influenza viruses

The *Orthomyxoviridae* (also referred to as influenzaviruses) are a family of RNA viruses that includes five genera: influenzavirus A, influenzavirus B, influenzavirus C, isavirus and thogotovirus. Influenzavirus A, influenzavirus B, influenzavirus C and thogotoviruses cause influenza in vertebrats, including humans. Isaviruses infect salmon. Viruses of this family contain 6 to 8 segments of linear negative-sense single stranded RNA. The total genome length is 12000-15000 nucleotides. The viruses of this family are very similar in structure and the envelope can occur in spherical and filamentous forms. In general, the virus's morphology is spherical with particles (virions) 50 to 120 nm in diameter, or filamentous virions 20 nm in diameter and 200 to 300 (-3000) nm long.

Although a number of drugs and methods exist or are currently under development for the treatment of viral infections, many of these are complicated by the side effects the anti-viral drug(s) has/have on the patient. Moreover, many of the known flavonoids have stability problems (oxidation, significant and rapid metabolism and conjugation during absorption in the body, poor bioavailability in the blood plasma) hampering their possible application as drugs (Jeremy P. E. Spencer, Metabolism of Tea Flavonoids in the Gastrointestinal Tract, J. Nutr. October 1, 2003 vol. 133 no. 10 3255S-3261S; C. Manach et al., Polyphenols: food sources and bioavailability, Am J Clin Nutr 2004;79:727-47). Additionally, drug-resistance and viral genotype-specific efficacy of these drugs necessitate alternative antiviral treatment options. Therefore, new therapeutics that eliminate chronic virus replication, prevent progression of the viral disease and/or preclude reinfection of the host are needed.

It is, therefore, an aim of the present invention to provide novel antiviral compounds that are preferably inhibitors of virus entry, preferably of enveloped viruses, such as flaviviruses (e.g. hepatitis C virus (HCV), dengue virus, west nile virus), human immunodeficiency virus (HIV), herpesviruses, vesicular stomatitis virus (VSV), or influenza viruses. Additionally, the invention relates to compositions for use in the treatment and/or prevention of a viral infection.

### Summary and Description of the Invention

The present invention was made in view of the prior art and the needs described above, and, therefore, the object of the present invention is to provide novel alternative means for treating and/or preventing viral infections, preferably viral infections with enveloped viruses, such as flaviviruses (e.g. hepatitis C virus (HCV), dengue virus, west nile virus), human immunodeficiency virus (HIV), herpesviruses, vesicular stomatitis virus (VSV), or influenza viruses.

Other objects of the present invention are to provide a pharmaceutical composition for use in the treatment and/or prevention of viral infections, preferably infections with enveloped viruses, to provide a combinatorial composition, comprising compounds for treating such viral infections.

These objects are solved by the subject matter of the attached claims.

These and other aspects of the present invention will become apparent upon reference to the following detailed description and definitions.

The inventors established that certain compounds prevent virus life cycle steps, e.g. HCV, VSV and HIV-1 life cycle steps, subsequent to virus binding and prior to viral RNA translation and RNA replication. The inventors showed that combined administration of certain compounds and cyclosporine A had a synergistic effect in inhibition of HCV infection.

The present description discloses a compound of the general formula (I): 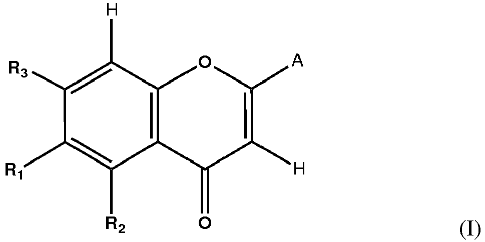 or a pharmacologically acceptable salt, solvate, or hydrate thereof, wherein
A is: 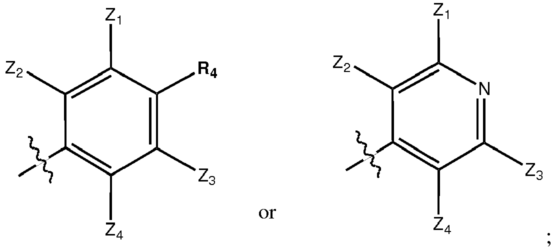 R₁ and R₂ are transient masked hydroxyl groups, cleavable under physiological conditions, taken together to form one of the following groups, like methylenedioxy, ethylenedioxy, diisopropylsilyl, diterbutylsilyl, acetonide, phenyl ketal, carbonate, sulfite, sulfate, phenyl O,O,O-orthoester, methyl O,O,O-orthoester, phenyl O,O,N-orthoester, methyl O,O,N-orthoester groups, as illustrated below: 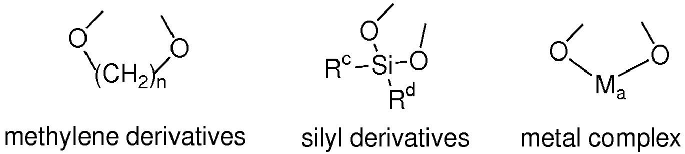 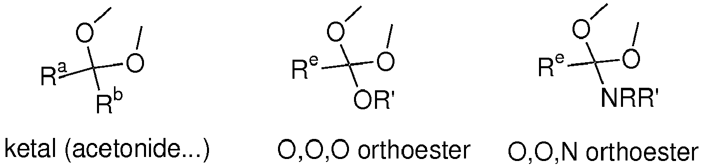 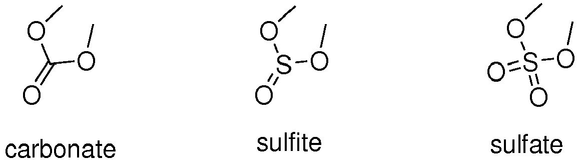 wherein
n is an integer of 1 to 3;
R^{a} and R^{b} each and independently of each other represent a hydrogen atom, halogen atom, cyano, nitro, an aryl, a C₁-C₆ alkyl or a C₁-C₆ alkoxy group;
R^{c} and R^{d} each and independently of each other represent a hydrogen atom, halogen atom, an aryl, a C₁-C₆ alkyl or a C₁-C₆ alkoxy group;
R^{e} represents a hydrogen atom, halogen atom, an aryl or a C₁-C₆ alkyl group;
R and R' each and independently of each other represent a hydrogen atom, a C₁-C₆ alkyl, or a C₂-C₆ alkenyl group;
Mₐ is a non electroactive metal ion under its divalent state such as zinc, magnesium, calcium;
   or
R₁ is a group OR₅ and R₂ is an oxygen atom that is taken together with the β-carbonyl unit to form a metal complex of the general formula (II): 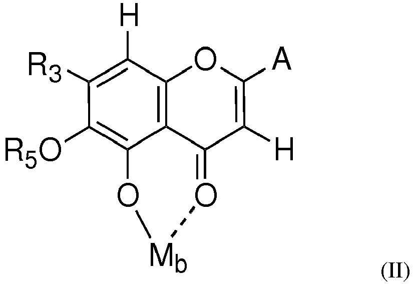 wherein
   M_{b} is a non electroactive metal ion under its divalent state such as zinc, magnesium, calcium, copper, or trivalent iron; and
   R₅ is a hydrogen atom or an acetyl group;
   R₃ is a C₁-C₆ alkyl or a C₁-C₆ alkoxy group;
   R₄ is a nitro, cyano, sulphur pentafluoride, -SO₃H, -SO₂NH₂, an optionally substituted C₁-C₆ alkyl or an optionally substituted C₁-C₆ alkoxy group, which may be substituted with one or more fluorine, chlorine, bromine or iodine atoms or by -OH, =O, -SH, =S, NH₂, =NH or -NO₂ group(s); and
   Z₁, Z₂, Z₃, and Z₄, each and independently of each other represent a hydrogen atom, halogen atom, cyano, nitro, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group.

The present invention is directed to a compound of the general formula (I): 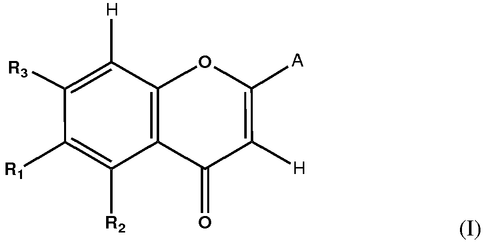 or a pharmacologically acceptable salt, solvate, or hydrate thereof, wherein
A is: 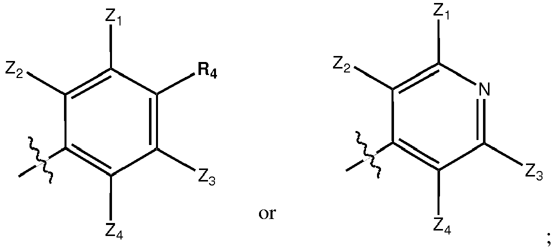 R₁ is a group OR₅ and R₂ is an oxygen atom that is taken together with the β-carbonyl unit to form a metal complex of the general formula (II): 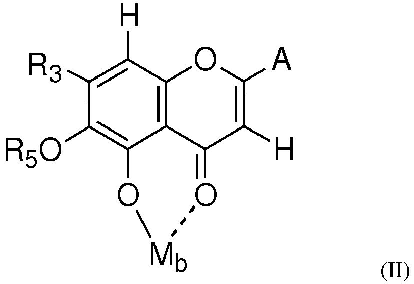 wherein
M_{b} is a non electroactive metal ion under its divalent state such as zinc, magnesium, calcium, copper, or trivalent iron; and
R₅ is a hydrogen atom or an acetyl group;
R₃ is a C₁-C₆ alkyl or a C₁-C₆ alkoxy group;
R₄ is a nitro, cyano, sulphur pentafluoride, -SO₃H, -SO₂NH₂, an optionally substituted C₁-C₆ alkyl or an optionally substituted C₁-C₆ alkoxy group, which may be substituted with one or more fluorine, chlorine, bromine or iodine atoms or by -OH, =O, -SH, =S, NH₂, =NH or -NO₂ group(s); and
Z₁, Z₂, Z₃, and Z₄, each and independently of each other represent a hydrogen atom, halogen atom, cyano, nitro, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
or a compound selected from:

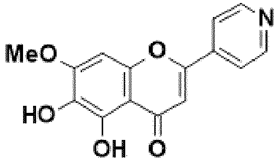

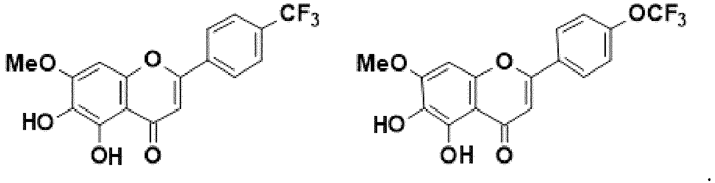

Compounds are generally described herein using standard nomenclature, unless otherwise specified, and may be obtained according to methods described in the literature (e.g. Flavone synthesis: Maiti, A.; Cuendet, M.; Kondratyuk, T.; Croy, V. L.; Pezzuto, J. M.; Cushman, M. Synthesis and Cancer Chemopreventive Activity of Zapotin, a Natural Product from Casimiroa edulis. J. Med. Chem. 2007, 50, 350-355; **Elbs oxidation for synthesis of the starting material 1:** Elbs K. Ueber Nitrohydrochinon. J. Prakt. Chem. 1893, 48, 179-185; **Methylenation of catechol :** A.P. Bashall and J.F. Collin, Tetrahedron Lett. 1975, No. 40, 3489-3490; **Silyl protection of catechol** : Y. Chung, B. F. Duerr, T. A. McKelvey, P. Nanjappan, A. W. Czarnik J. Org Chem. 1989, 54, 1018-1032; **Acetonide formation from catechol:** M. Node, M. Ozeki, L. Planas, M. Nakano, H. Takita, D. Mori, S. Tamatani, T. Kajimotoor J. Org Chem. 2010, 75, 190-196; **Phenyl ketal formation from catechol**: S. R. K. Pingali, B. S. Jursic Tetrahedron Lett. 2011, 52, 4371-4374; ***O,O,O*-orthoester formation from catechol**: Gross, H.; Rusche, J. Chemische Berichte, 1966, 99, 2625-30; ***O,O,N*-orthoester formation from catechol**: Sokolova, E. A.; Maretina, I. A.; Petrov, A. A. Zhurnal Organicheskoi Khimii, 23(10), 2097-100; 1987; **Carbonate from catechol:** Hwu, Jih Ru; Hsu, Ming-Hua; Huang, Ru Chih C. Bioorg. Med. Chem. Lett. 2008, 18, 1884-1888; **Sulfate from catechol:** G. E. DuBois and R. A. Stephenson J. Org Chem. 1980, 45, 5371-5373; **Sulfite from catechol:** A. Adejare, D. D. Miller, J. S. Fedyna, C.-H Ahn, D. R. Feller J. Med. Chem. 1986, 29, 1603-1609; D. Malešev, V. Kunti, Investigation of metal-flavonoid chelates and the determination of flavonoids via metal-flavonoid complexing reactions, J. Serb. Chem. Soc. 72 (10) 921-939 (2007); Roshal A.D., Grigorovich A.V., Doroshenko A.O., Pivovarenko V.G., Demchenko A.P. Flavonols as metal-ion chelators: complex formation with Mg2+ and Ba2+ cations in the excited state. J. Photochem. Photobiol. A: Chem. 1999, 127, 89-100)

For compounds having asymmetric centers, it should be understood that, unless otherwise specified, all of the optical isomers and mixtures thereof are encompassed. Compounds with two or more asymmetric elements can also be present as mixtures of diastereoisomers. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather encompasses all tautomeric forms. Recited compounds further encompass compounds in which one or more atoms are replaced with an isotope, *i.e.,* an atom having the same atomic number but a different mass number. By way of general example, and without limitation, isotopes of hydrogen include tritium and deuterium and isotopes of carbon include ¹¹C, ¹³C, and ¹⁴C.

Compounds according to the formulas provided herein, which have one or more stereogenic center(s), have an enantiomeric excess of at least 50%. For example, such compounds may have an enantiomeric excess of at least 60%, 70%, 80%, 85%, 90%, 95%, or 98%. Some embodiments of the compounds have an enantiomeric excess of at least 99%. It will be apparent that single enantiomers (optically active forms) can be obtained by asymmetric synthesis, synthesis from optically pure precursors or by resolution of the racemates. Resolution of the racemates can be accomplished, for example, by conventional methods such as crystallization in the presence of a resolving agent, or chromatography, using, for example, a chiral HPLC column.

Compounds herein may also be described using a general formula that includes variables such as, e.g. R₁-R₄, R^{a}-R^{e}, Z₁-Z₄, Mₐ, M_{b} etc. Unless otherwise specified, each variable within such a formula is defined independently of any other variable, and any variable that occurs more than one time in a formula is defined independently of each occurrence. Thus, for example, if a group is shown to be substituted with 0-2 R*, the group may be unsubstituted or substituted with up to two R* groups and R* at each occurrence is selected independently from the definition of R*. Also, combinations of substituents and/or variables are permissible only if such combinations result in stable compounds, i.e. compounds that can be isolated, characterized and tested for biological activity.

A "pharmaceutically acceptable salt" of a compound disclosed herein preferably is an acid or base salt that is generally considered in the art to be suitable for use in contact with the tissues of human beings or animals without excessive toxicity or carcinogenicity, and preferably without irritation, allergic response, or other problem or complication. Such salts include inorganic and organic acid salts of basic residues such as amines or pyridines, as well as alkali or organic salts of acidic residues such as carboxylic acids or basic residues such as phenols or catechols, thiophenols.

Suitable pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to 4, *i.e.,* 0, 1, 2, 3, or 4, and the like. Similarly, pharmaceutically acceptable cations include, but are not limited to sodium, potassium, calcium, zinc, magnesium, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of non-aqueous media, such as ether, ethyl acetate, ethanol, isopropanol or acetonitrile, is preferred.

It will be apparent that each compound of formula (I) may, but need not, be present as a hydrate, solvate or non-covalent complex. In addition, the various crystal forms and polymorphs are within the scope of the present invention.

A "substituent," as used herein, refers to a molecular moiety that is covalently bonded to an atom within a molecule of interest, e.g. to a compound of formula (I) or a prodrug thereof. For example, a "ring substituent" may be a moiety such as a halogen atom, alkyl group, haloalkyl group or other substituent described herein that is covalently bonded to an atom, preferably a carbon or nitrogen atom, that is a ring member. The term "substituted," as used herein, means that any one or more hydrogens on the designated atom is replaced with a selection from the indicated substituents, provided that the designated atom's normal valence is not exceeded, and that the substitution results in a stable compound, *i.e.,* a compound that can be isolated, characterized and tested for biological activity. When a substituent is oxo, *i.e.,* =O, then 2 hydrogens on the atom are replaced. An oxo group that is a substituent of an aromatic carbon atom results in a conversion of -CH- to -C(=O)- and a loss of aromaticity. For example a pyridyl group substituted by oxo is a pyridone.

The expression alkyl preferably refers to a saturated, straight-chain or branched hydrocarbon group that contains from 1 to 20 carbon atoms, preferably from 1 to 12 carbon atoms, more preferably from 1 to 6 carbon atoms, for example a methyl, ethyl, propyl, iso-propyl, n-butyl, iso-butyl, sec-butyl, *tert-*butyl, n-pentyl, n-hexyl, n-heptyl, 2,2-dimethylbutyl, n-octyl, 2,2,4-trimethylpentyl, nonyl, decyl, undecyl, or dodecyl group.

The expressions alkenyl and alkynyl refer to at least partially unsaturated, straight-chain or branched hydrocarbon groups that contain from 2 to 20 carbon atoms, preferably from 2 to 12 carbon atoms, more preferably from 2 to 6 carbon atoms, for example an ethenyl, allyl, acetylenyl, propargyl, isoprenyl or hex-2-enyl group. Preferably, alkenyl groups have one or two, more preferably one, double bond(s) and alkynyl groups have one or two, more preferably one, triple bond(s).

The expression heteroalkyl preferably refers to an alkyl, alkenyl or alkynyl group, for example heteroalkenyl, heteroalkynyl, in which one or more, preferably 1, 2 or 3 carbon atoms have been replaced each independently of the others by an oxygen, nitrogen, phosphorus, boron, selenium, silicon or sulphur atom, preferably oxygen, sulphur or nitrogen. The expression heteroalkyl, for example, encompasses an alkoxy group. An alkoxy group denotes an alkyl group linked to oxygen thus: -O-alkyl. Furthermore, heteroalkyl preferably refers to a carboxylic acid or to a group derived from a carboxylic acid such as, for example, acyl, acylalkyl, alkoxycarbonyl, acyloxy, acyloxyalkyl, carboxyalkylamide, alkylcarbamoylalkyl, alkylcarbamoyloxyalkyl, alkylureidoalkyl, or alkoxycarbonyloxy.

Examples of heteroalkyl groups are groups of formulas -S-Y^{a'}-L, -S-Y^{a'}-CO-NR^{a'}R^{b'}, -Y^{a'}-NR^{c'}-CO-NR^{a'}R^{b'}, -Y^{a'}-NR^{c'}-CO-O-R^{d'}, -Y^{a'}-NR^{c'}-CO-R^{d'}, -Y^{a'}-NR^{c'}-CO-NR^{d'}-L, -Y^{a'}-NR^{c'}-CS-NR^{d'}-L, -Y^{a'}-O-CO-NR^{a'}R^{b'}, -Y^{a'}-CO-NR^{a'}R^{b'}, -O-Y^{a'}-CO-NR^{a'}R^{b'}, -Y^{a'}-NR^{c'}-CO-L, -Y^{a'}-O-CO-O-R^{c'}. -Y^{a'}-O-CO-R^{c'}, -Y^{a'}-O-R^{c'}, -Y^{a'}-CO-L, -Y^{a'}-NR^{a'}R^{b'}, R^{c'}-S-Y^{a'}-, R^{a'}-N(R^{b'})-Y^{a'}-, R^{c'}-CO-Y^{a'}-, R^{c'}-O-CO-Y^{a'}-, R^{c'}-CO-O-Y^{a'}-, R^{c'}-CO-N(R^{b'})-Y^{a'}-, R^{a'}-N(R^{b'})-CO-Y^{a'}-, R^{c'}-SO-Y^{a'}-, R^{c'}-SO₂-Y^{a'}-, -Y^{a'}-NR^{c'}-SO₂-NR^{a'}R^{b'}, -Y^{a'}-SO₂-NR^{a'}R^{b'}, -Y^{a'}-NR^{c'}-SO₂-R^{d'}, R^{a'}-O-CO-N(R^{b'})-Y^{a'}-, R^{a'}-N(R^{b'})-C(=NR^{d'})-N(R^{c'})-Y^{a'}-, R^{c'}-S-CO-Y^{a'}-, R^{c'}-CO-S-Y^{a'}-, R^{c'}-S-CO-N(R^{b'})-Y^{a'}-, R^{a'}-N(R^{b'})-CO-S-Y^{a'}-, R^{c'}-S-CO-O-Y^{a'}-, R^{c'}-O-CO-S-Y^{a'}-, R^{c'}-S-CO-S-Y^{a'}-; wherein R^{a'} being a hydrogen atom, a C₁-C₆alkyl, a C₂-C₆alkenyl, a C₂-C₆alkynyl , or is joined to R^{b'} to form a 4- to 10-membered cycloalkyl or heterocycloalkyl; R^{b'} being a hydrogen atom, a C₁-C₆alkyl, a C₂-C₆alkenyl or a C₂-C₆alkynyl , or taken together with R^{a'} to form a 4- to 10-membered cycloalkyl or heterocycloalkyl; R^{c'} being a hydrogen atom, an optionally substituted C₁-C₈alkyl, an optionally substituted C₂-C₈alkenyl or an optionally substituted C₂-C₈alkynyl ; R^{d'} being a hydrogen atom, optionally substituted C₁-C₈alkyl, optionally substituted C₂-C₈alkenyl or optionally substituted C₂-C₈alkynyl; L being a cycloalkyl, heterocycloalkyl, alkylcycloalkyl, heteroalkylcycloalkyl, optionally substituted aryl, optionally substituted heteroaryl, aralkyl, or heteroaralkyl; and Y^{a'} being a bond, a C₁-C₆alkylene, a C₂-C₆alkenylene or a C₂-C₆alkynylene group. Specific examples of heteroalkyl groups are methoxy, trifluoromethoxy, ethoxy, n-propyloxy, isopropyloxy, *tert*-butyloxy, methoxymethyl, ethoxymethyl, methoxyethyl, methyl amino, ethylamino, dimethylamino, diethylamino, isopropylethylamino, methylaminomethyl, ethylaminomethyl, diisopropylaminoethyl, enol ether, dimethylaminomethyl, dimethylaminoethyl, acetyl, propionyl, butyryloxy, acetyloxy, methoxycarbonyl, ethoxycarbonyl, isobutyrylamino-methyl, N-ethyl-N-methylcarbamoyl and N-methylcarbamoyl. Further examples of heteroalkyl groups are nitrile, isonitrile, cyanate, thiocyanate, isocyanate, isothiocyanate and alkylnitrile groups. An example of a heteroalkylene group is a group of formulas -CH₂CH(OH)- or -CONH-.

The expression cycloalkyl preferably refers to a saturated or partially unsaturated cyclic group that contains one or more rings, preferably 1 or 2, containing from 3 to 14 ring carbon atoms, preferably from 3 to 10, more preferably 3, 4, 5, 6 or 7, ring carbon atoms. In an embodiment a partially unsaturated cyclic group has one, two or more double bonds, such as a cycloalkenyl group. Examples of a cycloalkyl group are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetralin, cyclopentylcyclohexyl, fluorocyclohexyl or cyclohex-2-enyl group.

The expression heterocycloalkyl preferably refers to a cycloalkyl group as defined above in which one or more, preferably 1, 2 or 3, ring carbon atoms have been replaced each independently of the others by an oxygen, nitrogen, silicon, selenium, phosphorus or sulphur atom, preferably oxygen, sulphur or nitrogen. A heterocycloalkyl group has preferably 1 or 2 ring(s) containing from 3 to 10, more preferably 3, 4, 5, 6 or 7, ring atoms. Examples are a piperidyl, piperazinyl, morpholinyl, urotropinyl, pyrrolidinyl, tetrahydro-thiophenyl, tetrahydropyranyl, tetrahydrofuryl or 2-pyrazolinyl group and also a lactam, a lactone, a cyclic imide and a cyclic anhydride.

The expression alkylcycloalkyl preferably refers to a group containing both cycloalkyl and also an alkyl, alkenyl or alkynyl group in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two ring systems having from 3 to 10, preferably 3, 4, 5, 6 or 7, carbon atoms, and one or two alkyl, alkenyl or alkynyl groups having 1 or 2 to 6 carbon atoms, the cyclic groups being optionally substituted.

The expression heteroalkylcycloalkyl preferably refers to alkylcycloalkyl groups as defined above in which one or more, preferably 1, 2 or 3, carbon atoms have been replaced each independently of the others by an oxygen, nitrogen, silicon, selenium, phosphorus or sulphur atom, preferably oxygen, sulphur or nitrogen. A heteroalkylcycloalkyl group preferably contains 1 or 2 ring systems having from 3 to 10, preferably 3, 4, 5, 6 or 7, ring atoms, and one or two alkyl, alkenyl, alkynyl or heteroalkyl groups having from 1 or 2 to 6 carbon atoms. Examples of such groups are alkylheterocycloalkyl, heterocycloalkylalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being optionally substituted and saturated or mono-, di- or tri-unsaturated.

The expression aryl or Ar preferably refers to an aromatic group that contains one or more rings containing from 6 to 14 ring carbon atoms, preferably from 6 to 10, more preferably 6, ring carbon atoms. Examples are a phenyl, naphthyl, biphenyl, or anilinyl group.

The expression heteroaryl preferably refers to an aromatic group that contains one or more rings containing from 5 to 14 ring atoms, preferably from 5 to 10, more preferably 5 or 6, ring atoms, and contains one or more, preferably 1, 2, 3 or 4, oxygen, nitrogen, phosphorus or sulphur ring atoms, preferably O, S or N. Examples are 4-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl.

The expression aralkyl preferably refers to a group containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl, 1H-indene, tetralin, dihydronaphthalene, phenylcyclopentyl, cumene, cyclohexylphenyl, fluorene and indan. An aralkyl group preferably contains one or two aromatic ring systems, 1 or 2 rings, containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl preferably refers to an aralkyl group as defined above in which one or more, preferably 1, 2, 3 or 4, carbon atoms have been replaced each independently of the others by an oxygen, nitrogen, silicon, selenium, phosphorus, boron or sulphur atom, preferably oxygen, sulphur or nitrogen, that is to say to groups containing both aryl or heteroaryl and also alkyl, alkenyl, alkynyl and/or heteroalkyl and/or cycloalkyl and/or heterocycloalkyl groups in accordance with the above definitions. A heteroaralkyl group preferably contains one or two aromatic ring systems, 1 or 2 rings, containing from 5 or 6 to 10 ring carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced each independently of the others by oxygen, sulphur or nitrogen atoms.

Examples of heteroaralkyl groups are aryloxy, arylheteroalkyl, arylheterocycloalkyl, arylheterocyclo-alkenyl, arylalkylheterocycloalkyl, arylalkenylheterocycloalkyl, arylalkynylheterocycloalkyl, arylalkyl-heterocycloalkenyl, heteroarylalkyl, heteroarylalkenyl, heteroarylalkynyl, heteroarylheteroalkyl, hetero-arylcycloalkyl, heteroarylcycloalkenyl, heteroarylheterocycloalkyl, heteroarylheterocycloalkenyl, hetero-arylalkylcycloalkyl, heteroarylalkylheterocycloalkenyl, heteroarylheteroalkylcycloalkyl, heteroarylhetero-alkylcycloalkenyl, heteroalkylheteroarylalkyl and heteroarylheteroalkylheterocycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

The expression "substituted" or "optionally substituted" as used herein in connection with any group refers to a group in which one or more hydrogen atoms have been replaced each independently of the others by fluorine, chlorine, bromine or iodine atoms or by -OH, =O, -SH, =S, -NH₂, =NH, -CN or -NO₂ group(s). Examples of substituted alkyl are 2,2,2-trichloroethyl or trifluoromethyl, and examples of substituted aryl (or substituted Ar) are 2-fluorophenyl, 3-nitrophenyl or 4-hydroxyphenyl.

The expression "halogen" or "halogen atom" as preferably used herein means fluorine, chlorine, bromine, iodine.

As used herein a wording defining the limits of a range of length such as, e. g., "from 1 to 5" means any integer from 1 to 5, i. e. 1, 2, 3, 4 and 5. In other words, any range defined by two integers explicitly mentioned is meant to comprise and disclose any integer defining said limits and any integer comprised in said range.

The present description preferably discloses a compound of the general formula (I): 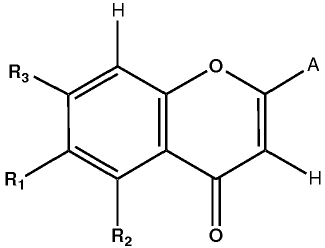 or a pharmacologically acceptable salt, solvate, or hydrate thereof, wherein
A is: 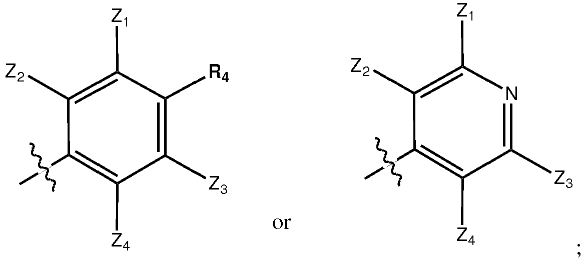 R₁ and R₂ are taken together to form a group: 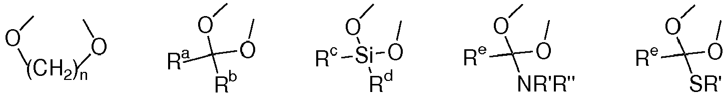 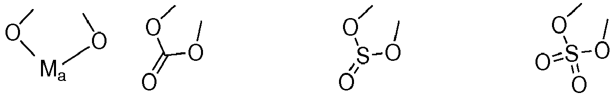 wherein
n is an integer of 1 to 3;
Mₐ represents Ca²⁺, Zn²⁺, or Mg²⁺;
R^{a} and R^{b} each and independently of each other represent a hydrogen atom, halogen atom, cyano, nitro, an aryl, a C₁-C₆ alkyl or a C₁-C₆ alkoxy group;
R^{c} and R^{d} each and independently of each other represent a hydrogen atom, halogen atom, an aryl, a C₁-C₆ alkyl or a C₁-C₆ alkoxy group;
R^{e} represents a hydrogen atom, halogen atom, an aryl or a C₁-C₆ alkyl group;
R' and R" each and independently of each other represent a hydrogen atom, a C₁-C₆ alkyl, or a C₂-C₆ alkenyl group;
R₃ is a C₁-C₆ alkyl or a C₁-C₆ alkoxy group;
R₄ is a nitro, cyano, sulphur pentafluoride, -SO₃H, -SO₂NH₂, an optionally substituted C₁-C₆ alkyl or an optionally substituted C₁-C₆ alkoxy group, which may be substituted with one or more fluorine, chlorine, bromine or iodine atoms or by -OH, =O, -SH, =S, NH₂, =NH or -NO₂ group(s); and
Z₁, Z₂, Z₃, and Z₄, each and independently of each other represent a hydrogen atom, halogen atom, cyano, nitro, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group.

Also preferred in the present invention is a compound of the general formula (II): 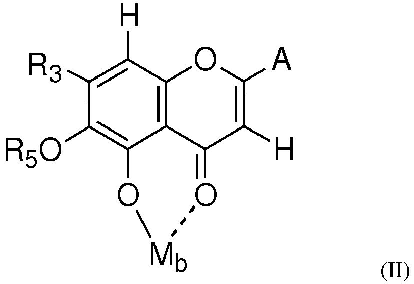 or a pharmacologically acceptable salt, solvate, or hydrate thereof, wherein
M_{b} represents Ca²⁺, Zn²⁺, Mg²⁺, or Fe³⁺; R₅ is a hydrogen atom or an acetyl group; and R₃ and A are defined as in the general formula (I).

Further preferred is a compound or salt of formula (I) or (II), wherein A is

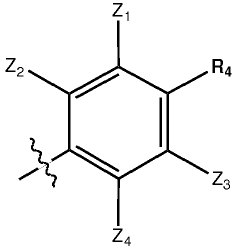

Also preferred is a compound or salt of formula (I) or (II), wherein A is:

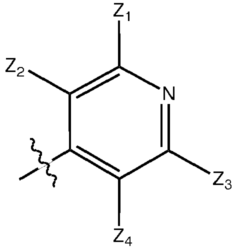

The present description also discloses a compound of formula (I), wherein R₁ and R₂ are taken together to form a methylenedioxy group:

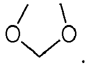

Preferably, in the compounds of the present invention, R₄ is OMe, CF₃, OCF₃, fluorine, chlorine, bromine, or -SO₃H. Further preferred, R₄ is OMe, CF₃, or OCF₃.

Further preferably, in the compounds of the present invention, R₃ is methyl or OMe, more preferably OMe.

Further preferably, in the compounds of the present invention, Z₁ to Z₄ are each and independently selected from hydrogen atom, fluorine, chlorine, bromine, and C₃-C₆ alkyl. Preferably, all of Z₁ to Z₄ represent hydrogen atoms.

Preferably, in the present description, R^{a}, R^{b}, R^{c}, R^{d}, and R^{e} each and independently of each other represent a hydrogen atom, an aryl or a C₁-C₆ alkyl group. Further preferred, R^{a}, R^{b}, R^{c}, R^{d}, and R^{e} are each and independently of each other hydrogen atom or a C₁-C₆ alkyl group.

The therapeutic use of compounds of general formula (I) or (II), their pharmacologically acceptable salts or solvates and hydrates also lie within the scope of the present invention, especially, their use as antiviral agents, especially for the treatment and/or prevention of an infection by an enveloped virus, such as flaviviruses (e.g. hepatitis C virus (HCV), dengue virus, west nile virus), human immunodeficiency virus (HIV), herpesviruses, vesicular stomatitis virus (VSV), or influenza viruses, particularly the treatment of hepatitis C.

The present invention also relates to a compound selected from: 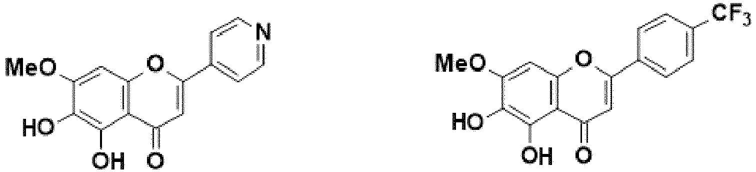 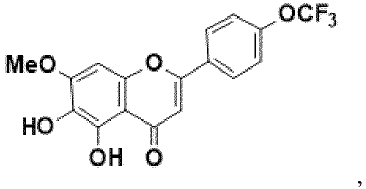 or a compound of formula: 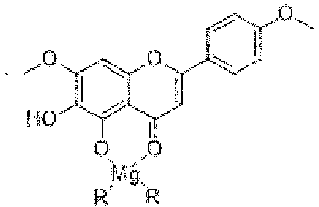 wherein R is stearate or Cl,
the 5,6-Dihydroxy-7-methoxy-2-(4-methoxy-phenyI)-chromen-4-one (BJ486K):Fe(III) complex and the 5,6-Dihydroxy-7-methoxy-2-(4-methoxy-phenyI)-chromen-4-one:Fe(III):NTA complex.

The present invention also relates to a pharmaceutical composition comprising at least one above-mentioned compound, and, optionally, at least one pharmaceutically acceptable carrier(s) and/or at least one customary excipient.

Preferably, the pharmaceutical composition of the invention comprises at least one compound selected from: 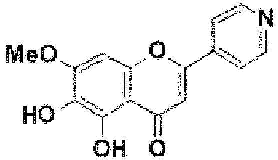 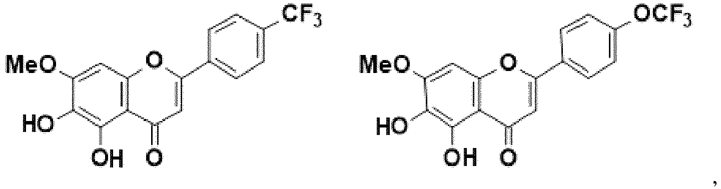 or one compound of formula: 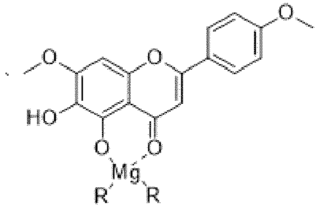 wherein R is stearate or Cl,
or one compound selected from the 5,6-Dihydroxy-7-methoxy-2-(4-methoxy-phenyI)-chromen-4-one:Fe(III) complex and the 5,6-Dihydroxy-7-methoxy-2-(4-methoxy-phenyI)-chromen-4-one:Fe(III):NTA complex.

Preferably, the compound used for the prevention and/or treatment of a viral infection is a compound according to general formula (I), (II), or a compound selected from: 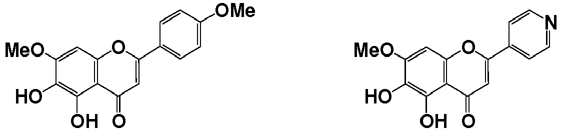 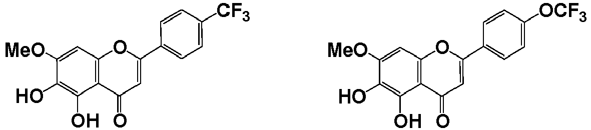 or a compound of formula: 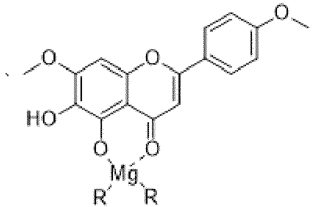 wherein R is stearate or Cl,
or is selected from the 5,6-Dihydroxy-7-methoxy-2-(4-methoxy-phenyI)-chromen-4-one:Fe(III) complex and the 5,6-Dihydroxy-7-methoxy-2-(4-methoxy-phenyI)-chromen-4-one:Fe(III):NTA complex.

Especially preferred according to the invention is the use of the compound:

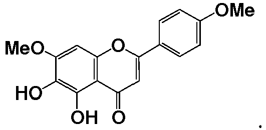

According to the invention, it is also possible to use a combination of compounds according to the invention.

The invention is also directed to the use of a compound according to general formula (I) or (II) for the preparation of a medicament intended for the prevention and/or treatment of a viral infection, preferably a viral infection by enveloped virus particles.

The invention also relates to an above-mentioned compound for use in the prevention and/or treatment of a viral infection, wherein the infection is an infection by an enveloped virus.

Moreover, the description is also directed to a pharmaceutical composition for use in the treatment and/or prevention of a viral infection, preferably a viral infection by enveloped virus particles, which pharmaceutical composition comprises at least one compounds according to general formula (I), (II), or a compound selected from: 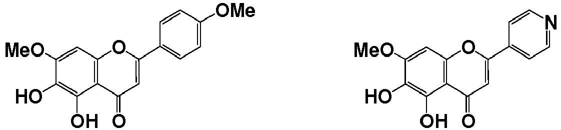 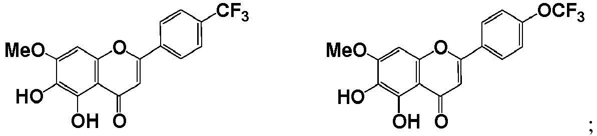 and, optionally, at least one pharmaceutically acceptable carrier(s) and/or at least one customary excipient.

By "treatment and/or prevention" is preferably meant a reduction or complete inhibiton of viral replication and/or infectivity in a subject to thereby cure or prevent the symptoms associated with the viral infection.

By inhibitor is meant any chemical or biological, natural or synthetic molecule, any composition which, whatever the mechanism, causes after administration a reduction, or even a complete inhibition, of the activity of the respective protein or member of the protein family or the expression of the corresponding gene(s) thereof. An inhibitor may either bind reversible or irreversible to its substrate. Reversible inhibitors comprise competitive inhibitors, non-competitive inhibitors, mixed-type inhibitors, uncompetitive inhibitors, slow-binding or tight-binding inhibitors, transition state analogs and multi-substrate analogs. A compound of the present invention may also be referred to as virus life cycle inhibitor, i.e. a compound that hampers or suppresses the initial interactions between a virus and the host cell that are essential for any virus infection.

The term "at least one" as used herein is intended to mean one, 2, or more of the respective item or subject.

Carrier substances are, for example, cyclodextrins such as hydroxypropyl β-cyclodextrin, micelles or liposomes, excipients and/or adjuvants. Pharmaceutical compositions may additionally comprise, for example, one or more of water, buffers such as, *e.g.,* neutral buffered saline or phosphate buffered saline, ethanol, propylene glycol, mineral oil, vegetable oil, dimethylsulfoxide, lipids, emulsifiers, carbohydrates such as *e.g.,* glucose, mannose, sucrose or dextrans, mannitol, proteins, adjuvants, polypeptides or amino acids such as glycine, antioxidants, metal ions such as zinc(II), calcium(II) or magnesium(II) and/or preservatives. Furthermore, one or more other active ingredients may, but need not, be included in the pharmaceutical compositions provided herein. For instance, the compounds of the invention may advantageously be employed in combination with an antibiotic, anti-fungal, or anti-viral agent, an antihistamine, a non-steroidal anti-inflammatory drug, a disease modifying anti-rheumatic drug, a cytostatic drug, a drug with smooth muscle activity modulatory activity or mixtures of the aforementioned.

S. Sholz, G. Williamson, Interactions Affecting the Bioavailability of Dietary Polyphenols in Vivo, Int. J. Vitam. Nutr. Res. 77, 2007, 224-235.

Pharmaceutical compositions may be formulated for any appropriate route of administration, including, for example, topical such as, *e.g.,* transdermal or ocular, oral, buccal, nasal, vaginal, rectal or parenteral administration. The term parenteral as used herein includes subcutaneous, intradermal, intravascular such as, *e.g.,* intravenous, intramuscular, spinal, intracranial, intrathecal, intraocular, periocular, intraorbital, intrasynovial and intraperitoneal injection, as well as any similar injection or infusion technique. In certain embodiments, compositions in a form suitable for oral use are preferred. Such forms include, for example, tablets, troches, lozenges, aqueous or oily suspensions, dispersible powders or granules, emulsion, hard or soft capsules, or syrups or elixirs. Within yet other embodiments, compositions provided herein may be formulated as a lyophilizate.

Compositions intended for oral use may further comprise one or more components such as sweetening agents, flavoring agents, coloring agents and/or preserving agents in order to provide appealing and palatable preparations. Tablets contain the active ingredient in admixture with customary (physiologically acceptable) excipients that are suitable for the manufacture of tablets.

Customary excipients include, for example, inert diluents such as, *e.g.,* calcium carbonate, sodium carbonate, lactose, calcium phosphate or sodium phosphate, granulating and disintegrating agents such as, *e.g.,* corn starch or alginic acid, binding agents such as, *e.g.,* starch, gelatin or acacia, and lubricating agents such as, *e.g.,* magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monosterate or glyceryl distearate may be employed.

The pharmaceutical composition of the invention may comprise at least one of the above virus life cycle inhibitors. The pharmaceutical composition may also comprise 2 or more of the above virus life cycle inhibitors.

For the treatment and/or prevention of a viral infection, preferably an infection by an enveloped virus particle, the dose of the biologically active compound according to the invention may vary within wide limits and may be adjusted to individual requirements. Active compounds according to the present invention are generally administered in a therapeutically effective amount. Preferred doses range from about 0.1 mg to about 140 mg per kilogram of body weight per day, and/or about 5 mg to about 7 g per patient per day. The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

According to the invention, the above virus life cycle inhibitor(s) can be used in the pharmaceutical preparation in a quantity comprised between 0.01 mg and 2 g, preferably from 1 mg to 1 g, very preferably from 10 mg to 500 mg.

More particularly, the above virus life cycle inhibitor(s) can in particular be administered in doses comprised between 0.1 mg/kg and 500 mg/kg, preferably 1 mg/kg and 100 mg/kg, very preferably 10 mg/kg and 50 mg/kg.

It will be understood, however, that the specific dose level for any particular patient will depend upon a variety of factors including the activity of the specific compound employed, the age, body weight, general health, sex, diet, time of administration, route of administration, and rate of excretion, drug combination, *i.e.* other drugs being used to treat the patient, and the severity of the particular disease undergoing therapy.

The invention is also related to a combination preparation containing at least one of the above virus life cycle inhibitors and at least one further active pharmaceutical ingredient for the treatment or prophylaxis of viral infections, such as an HCV, HIV, VSV, herpes or influenza infection.

The present invention also relates to a combination preparation for use in the treatment or prophylaxis of a viral infection containing at least one above-mentioned compound, or the compound: 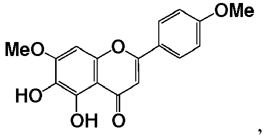 or a pharmacologically acceptable salt, solvate, or hydrate thereof, and at least one further active pharmaceutical ingredient.

The present invention also relates to a combination preparation for use in the treatment or prophylaxis of a viral infection containing at least one compound selected from: 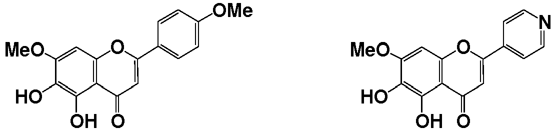 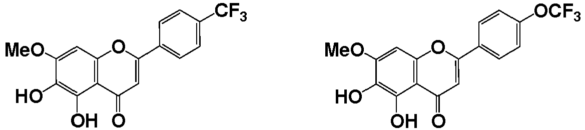 or one compound of formula: 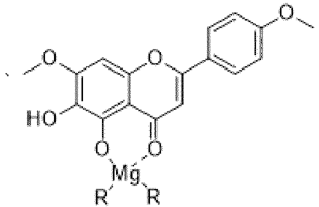 wherein R is stearate or Cl,
or one compound selected from the 5,6-Dihydroxy-7-methoxy-2-(4-methoxy-phenyI)-chromen-4-one:Fe(III) complex and the 5,6-Dihydroxy-7-methoxy-2-(4-methoxy-phenyI)-chromen-4-one:Fe(III):NTA complex.

Preferably, in the combination preparation of the invention the further active pharmaceutical ingredient is selected from cyclosporine A, pegylated interferon-alpha-2a, pegylated interferon-alpha-2b, ribavirin, viramidine, boceprevir (Victrelis), telaprevir (Incivo), *SP 30, ITX 5061, RG7128, PSI-7977, NM 283, Albuferon* and/or *Zadaxin,* most preferably cyclosporine A.

Moreover, it is preferred that the combination preparation of the invention contains at least one inhibitor of cPLA2. Most preferred is a combination of at least one cPLA2 inhibitor and at least one of pegylated interferon-alpha-2a, pegylated interferon-alpha-2b, ribavirin and viramidine.

Preferably, the above virus life cycle inhibitors used for the treatment of viral infections will have certain pharmacological properties. Such properties include, but are not limited to oral bioavailability, such that the preferred oral dosage forms discussed above can provide therapeutically effective levels of the compound *in vivo.*

The above virus life cycle inhibitors are preferably administered to a patient orally or parenterally, and are present within at least one body fluid or tissue of the patient.

Accordingly, the present disclosure further provides methods for preventing or treating patients suffering from a viral infection, especially an infection by an enveloped virus particle.
As used herein, the term "treatment" encompasses both disease-modifying treatment and symptomatic treatment, either of which may be prophylactic, i.e., before the onset of symptoms, in order to prevent, delay or reduce the severity of symptoms, or therapeutic, i.e., after the onset of symptoms, in order to reduce the severity and/or duration of symptoms.

Preferably, the method of preventing or treating a viral infection according to the present disclosure comprises administering to a subject in need thereof an effective amount of at least one of the above virus life cycle inhibitors.

In a preferred method of preventing or treating a HCV infection according to the present disclosure, the method comprises administration of at least one of the virus life cycle inhibitors detailed above for use in the prevention and/or treatment of HCV infection. More particularly, the method preferably comprises administration of at least one of the virus life cycle inhibitors:

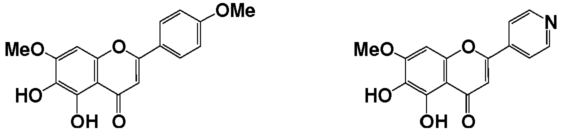

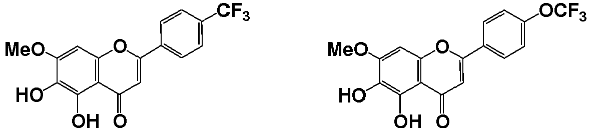

The method of preventing or treating a viral infection, e.g. HCV infection, according to the present disclosure may, moreover, be characterized in that the virus life cycle inhibitor is intended to be administered by oral route, by aerosol route or by injection.

It is especially preferred to combine the preferred embodiments of the present invention in any possible manner.

### Brief Description of the Figures

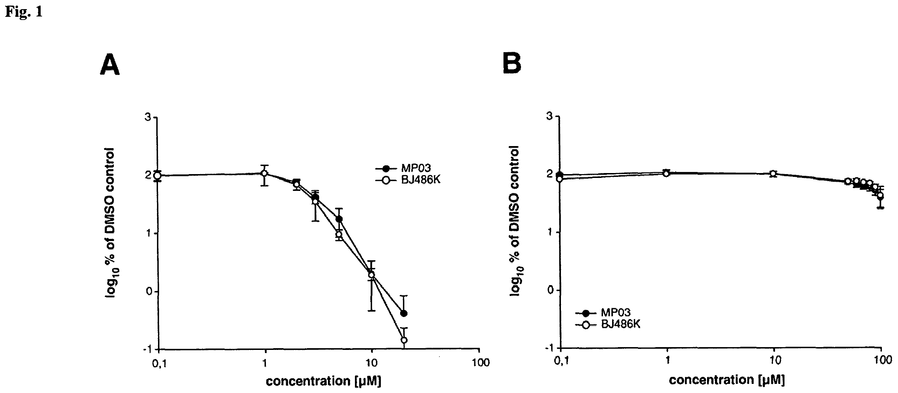

**Figure 1****: Antiviral activity of BJ486K.** (**A**) Huh7-Lunet/CD81 cells were inoculated for 48h with Luc-Jc1 virus particles in the presence of increasing doses of BJ486K or MP03, respectively. HCV infection and replication efficiency was determined 48h post inoculation using luciferase assays and is expressed as % of cells treated with solvent alone. Mean values of three independent experiments each with quadruplicate measurements including the standard deviation are shown. (**B**) Huh7-Lunet/CD81 cells were cultured for 48h in the presence of increasing doses of BJ486K or MP03, respectively. Cell viability was determined using a commercial cytotoxicity assay (CytoToxGlo; Promega, Germany) and is expressed relative to cells treated with DMSO. Mean values of three independent experiments each with quadruplicate measurements including the standard deviation are shown.

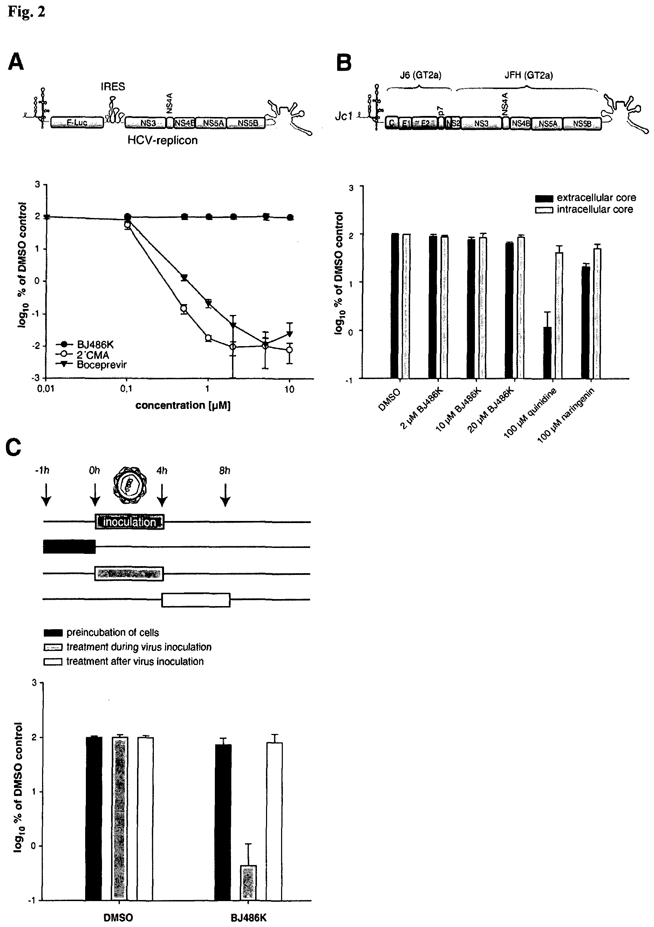

**Figure 2****: BJ486K selectively inhibits HCV cell entry.** (**A**) Huh7-Lunet/CD81 cells were transfected with the subgenomic luciferase replicon depicted at the top. Drugs were added 4h post transfection and the level of HCV RNA translation and RNA replication was quantified by luciferase assays 72h post transfection. Mean values of three independent experiments each with quadruplicate measurements including the standard deviation are shown. (**B**) Cells were transfected with the Jc1 chimera depicted above and drugs were added 4h later. HCV virus release in the presence or absence of given compounds was determined by quantification of intracellular and extracellular core protein levels using a core-specific ELISA. Mean values of three independent experiments with single measurements including the error range are given. (**C**) Huh7-Lunet/CD81 cells were inoculated with Luc-Jc1 reporter viruses prepared in the absence of drugs. BJ486K was added to the cells only prior to inoculation (black), only during inoculation (grey) or selectively directly after inoculation (white) as schematically depicted at the top. Infectivity was determined 72h later by luciferase assays and compared to DMSO-treated controls. Mean values of three independent experiments each with quadruplicate measurements including the standard deviation are shown.

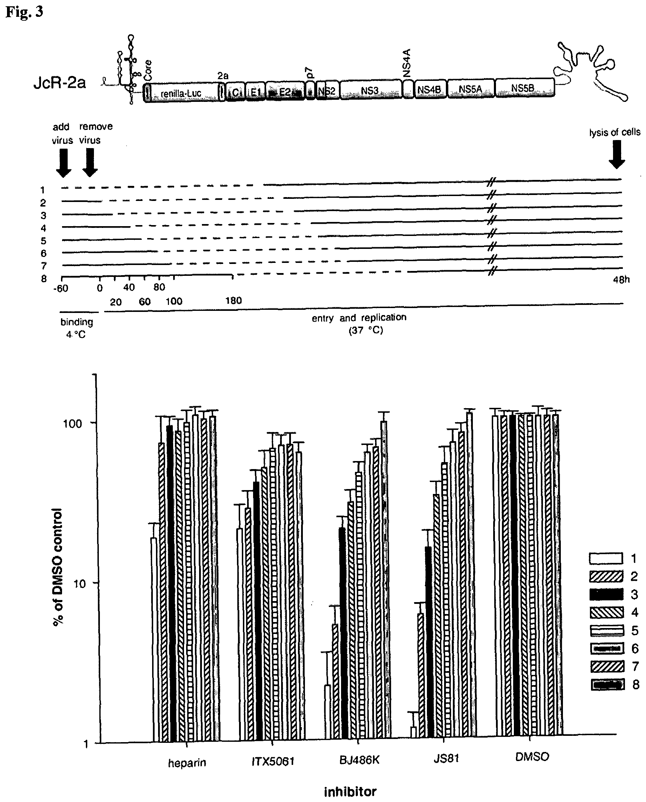

**Figure 3****: BJ486K inhibits cell entry post virus attachment.**
   The design of the JcR-2a reporter virus construct is given at the top. Huh7-Lunet/CD81 cells were inoculated with concentrated JcR-2a particles at 4°C for 60 minutes. Subsequently, unbound virus particles were washed away and cells were shifted to 37°C to permit synchronized cell entry. According to the 8 experimental protocols depicted in the middle, various cell entry inhibitors including heparin (50 µg/ml), ITX5061 (10 µM), CD81-specific antibody JS-81 (2 µg/ml) and BJ486K (20 µM) were either present during inoculation (protocol 1) or were added at different time points after inoculation (protocols 2 to 8). In each case, inhibitor was present for 4h (dashed line) and was then washed away. Efficiency of cell entry was determined 48h post inoculation by determination of luciferase activity and is expressed relative to control infections conducted in the presence of solvent alone (DMSO). Mean values of three independent experiments each with quadruplicate measurements including the standard deviation are shown.

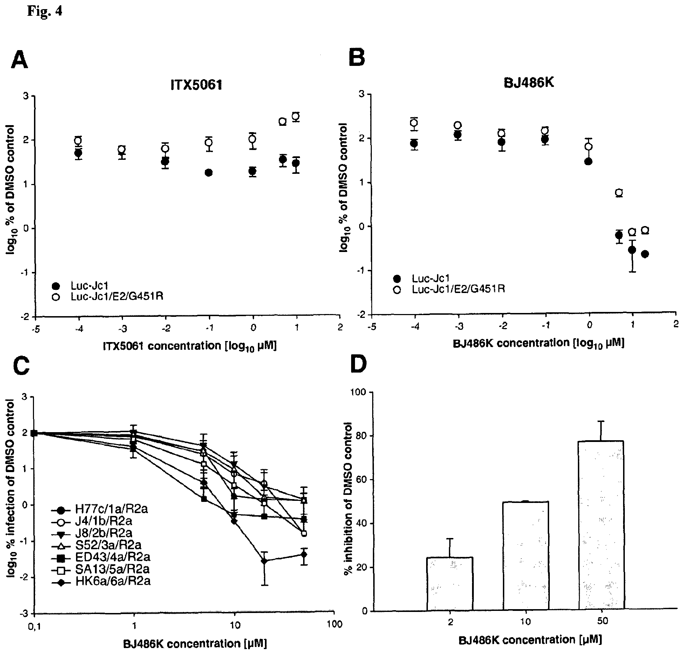

**Figure 4****: BJ486K is not affected by viral resistance towards a SR-BI-targeting compound, inhibits HCV of different genotypes and prevents infection of primary human hepatocytes** Huh7-Lunet/CD81 cells were inoculated with Luc-Jc1 or Luc-Jc1/E2/G451R reporter viruses in the presence of increasing doses of (A) ITX 5061 or (B) BJ486K. Cells were lysed 72h later. Luciferase activity was determined and is expressed relative to control infections conducted in the presence of DMSO. Mean values of four independent experiments each with quadruplicate measurements including the standard deviation are shown. (C) Huh7-Lunet/CD81 cells were inoculated with genotype 1a, 1b, 2b, 3a, 4a, 5a and 6a chimeric reporter viruses in the presence of increasing doses of BJ486K. Luciferase activity was determined 72h later and is expressed relative to infections in the presence of DMSO. Mean values of three independent experiments, each with triplicate measurements, are given. (D) Primary human hepatocytes were inoculated with Con1/C3 in the presence of DMSO or increasing concentrations of BJ486K. The inhibition of HCV infection was assessed by focus-formation assay and is expressed as % inhibition relative to DMSO-treated cells. Mean values of duplicate measurements including the error range are given.

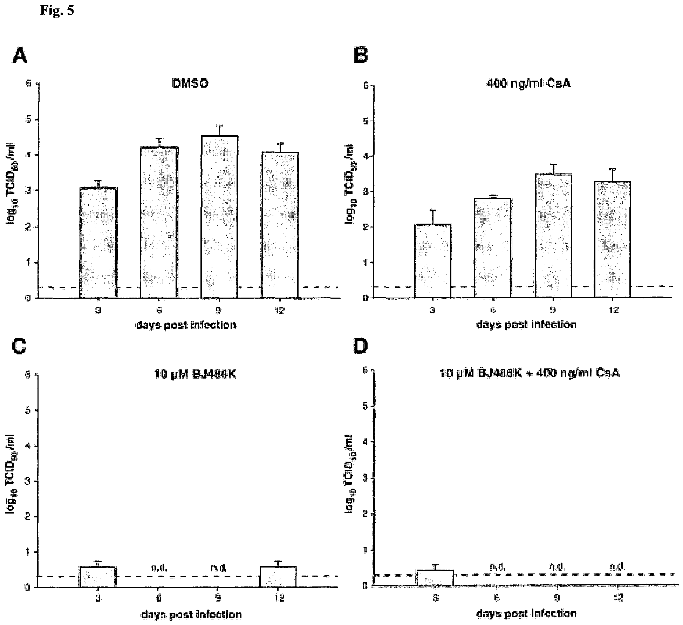

**Figure 5****: Combination of BJ486K and CsA prevents infection of differentiated human hepatoma cells.** Huh7-Lunet/CD81 cells were differentiated by treatment with 1% DMSO as described recently (Sainz B et al., J. Virol. 2006; 80: 10253-10257). Subsequently cells were challenged with Jc1 in the presence of DMSO (A), CsA (B), BJ486K (C), or a combination of CsA and BJ486K (D). Culture fluids of the inoculated cells were collected at 3, 6, 9 and 12 days post inoculation and the infectivity of released virus particles was determined by a limiting dilution assay.

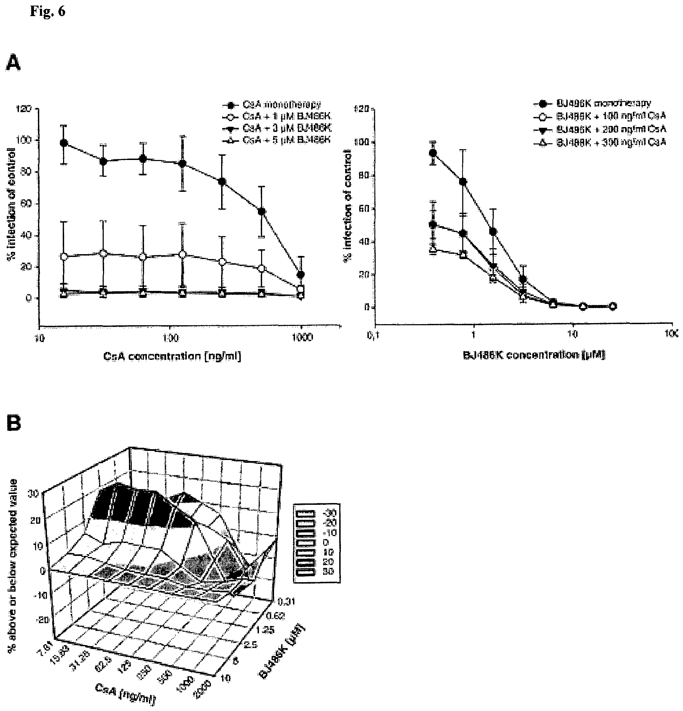

**Figure 6****: Synergistic antiviral activity of BJ486K and CsA.** Combined effects of CsA on BJ486K and vice versa were analysed by inoculation of Huh7-Lunet/CD81 cells with JcR-2a virus in the presence of given drugs and determination of luciferase activity 72h later. Mean values of three independent experiments each conducted in triplicates including the standard deviations are given. Data are normalized for values determined in mock treated controls.

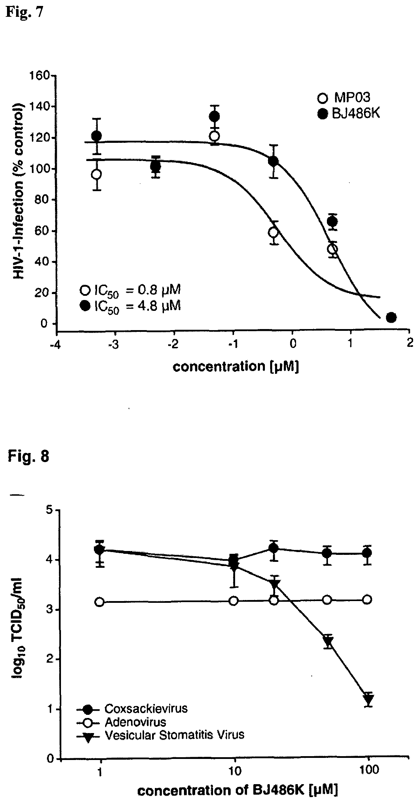

**Figure 7****: Antiviral activity of BJ486K on enveloped HIV virus.**
**Figure 8****: Antiviral activity of BJ486K on enveloped and non-enveloped viruses.** Influence of BJ486K on enveloped vesicular stomatitis viruses and non-enveloped group B coxsackievirus (CoxV) and adenovirus type 5 (AdV5); determined using limiting dilution assays.

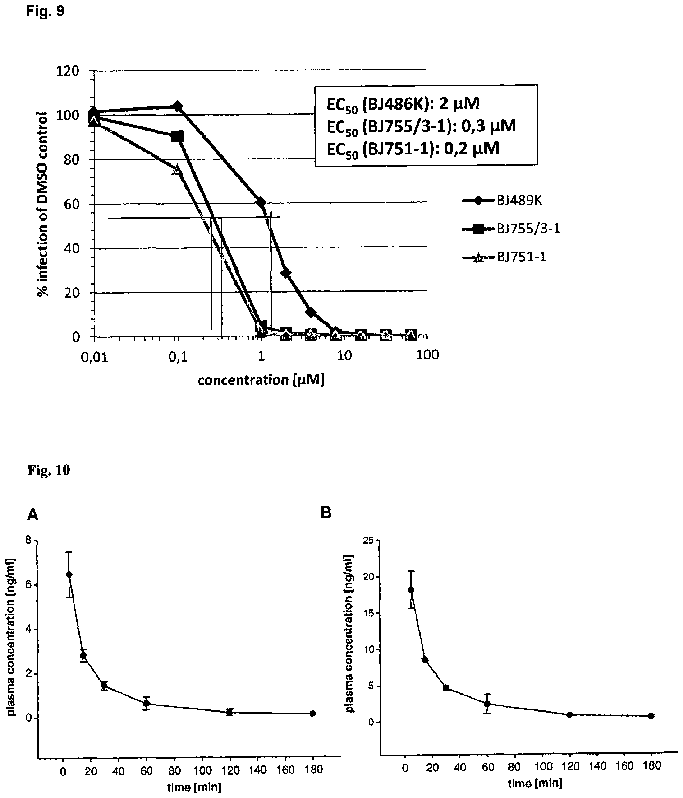

**Figure 9****: Comparison of flavones 13, 14 and 15.** Huh-7.5 cells were seeded in 12-well plates and infected for 4h with FF-Jc1 reporter virus +/- inhibitor (ranging from 0-100 µM final concentration). 72h after infection, the cells were lysed and the reporter activity measured. The mean values of 6 measurements are shown in the graph. Fluorine analogues of flavone 13, flavones 14 (BJ751/1) and 15 (BJ755-3 I), inhibit HCV cell entry with 8-10-fold increased effects compared to those of the flavone 13. Specifically, flavones 14 (BJ751/1) and 15 (BJ755-3 I) show an EC₅₀ of about 300 nM and an IC₉₀ of about 1 µM, while flavone 13 (BJ486K) shows an EC₅₀ of about 2000 nM.
**Figure 10****: Oral bioavailability of BJ486K in mice.** A single dose of 0.25mg/kg of BJ486K was administered through oral (A) or intravenous route (B). Plasma levels of BJ486K were determined 5, 15, 30, 60, 120 and 180 minutes post administration. Mean values of two animals including the standard errors are given. (C)

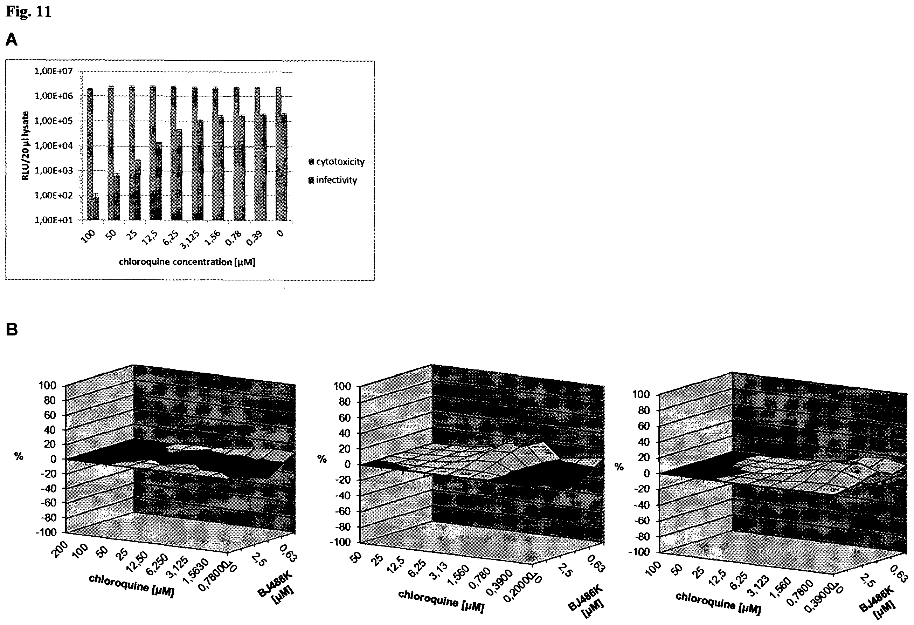

**Figure 11****: Additive antiviral activity of BJ486K and Chloroquine (CQ).** (A) Treatment with chloroquine alone shows a dose-dependent inhibition of HCV (red bar), whereas no cytotoxicity (blue bar) could be observed at the used concentrations. Result from one experiment with triplicate measurements is given. (B) Combined effects of CQ on BJ486K activity (and *vice versa*) were analyzed by inoculation of Huh7.5 F-Luc cells with JcR-2a virus in the presence of given drugs and determination of luciferase activity 72h later. Results from 3 independent experiments are given.

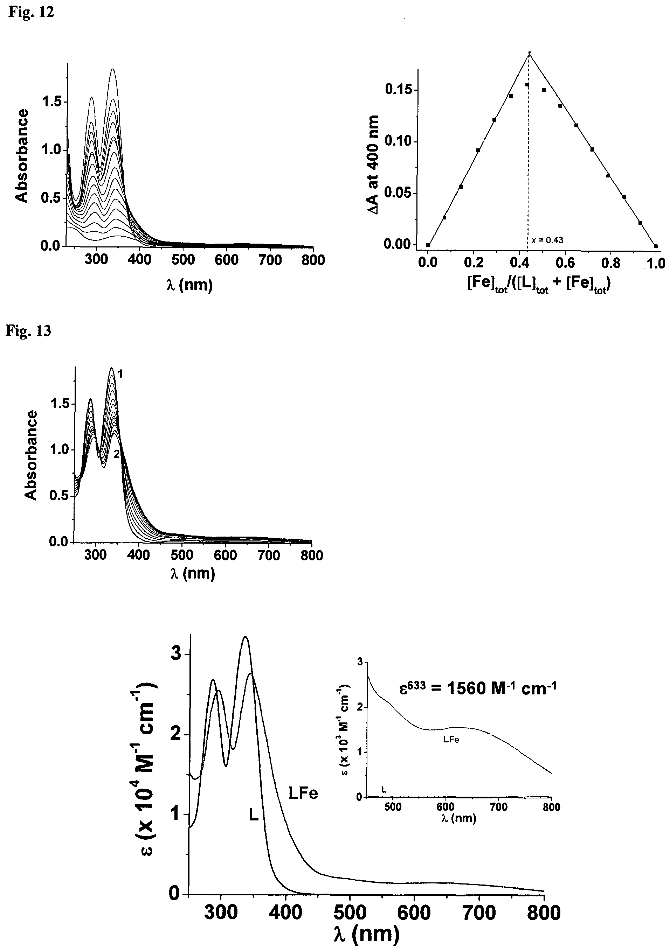

**Figure 12****: Determination of BJ486K/Fe(III) complex stoichiometry - Job's plot.** The stoichiometry of the BJ486K:Fe(III) complex was determined with the method of continuous variations (solvent: CH₃OH/H₂O (80/20 by weight); I = 0.1 M (NEt₄ClO₄); pH = 2.0; T = 25.0(2) °C; l = 1 cm; ([Fe(III)]ₜₒₜ + [BJ486K]ₜₒₜ) = 4.94 × 10⁻⁵ M). A series of solutions of **MB** and **Fe^{III}PPIX** at pH 2.0 subject to the condition that the sum of the total BJ486K and Fe(III) concentrations is constant (4.94 × 10⁻⁵ M) were prepared. Absorption spectra were measured for each of the solutions and the extremum xₘₐₓ value (∼ 0.43) indicates the predominant formation of a 1:1 stoichiometry complex (BJ486K: **Fe(III)).**
**Figure 13****: Spectrophotometric titration of BJ486K with Fe(III) solution at pH 2.0.** The absorption spectrophotometric titration of BJ486K by Fe(III) at pH 2.0 (solvent: CH₃OH/H₂O (80/20 by weight); I = 0.1 M (NEt₄ClO₄); T = 25.0(2) °C; pH = 2.0; 1 = 1 cm; (1) [BJ486K]ₜₒₜ = 5.92 × 10⁻⁵ M; (2) [Fe(III)]ₜₒₜ/[BJ486K]ₜₒₜ = 1.60) shows the ability of the flavone BJ486K to firmly bind Fe(III) through its β-hydroxy-ketone chelating unit. The Fe(III) coordination is evidenced by the formation of a BJ486K-to-Fe(III) charge transfer absorption band at 633 nm as typically observed for phenolate or catecholate Fe(III) binders ((a) D.D. Cox, S.J. Benkovic, L.M. Bloom, F.C. Bradley, M.J. Nelson, L. Que Jr., D.E. Wallick "Catecholate LMCT bands as probes for the active sites of nonheme iron oxygenases" J. Am. Chem. Soc. 1988, 110, 2026-2032. b) B.P. Gaber, V. Miskowski, T.G. Spiro "Resonance Raman scattering from iron(III)- and copper(II)-transferrin and an iron(III) model compound. Spectroscopic interpretation of the transferrin binding site" J. Am. Chem. Soc. 1974, 96, 6868-6873. c) M. Elhabiri, C. Carrër, F. Marmolle, H. Traboulsi "Complexation of iron(III) by catecholate-type polyphenols" Inorg. Chim. Acta 2007, 360, 353-359). The BJ486K:Fe(III) complex was characterized and quantified (log *K*^{*}_{BJ486K:Fe} = 6.0(3) at pH 2.0; *K*_{D} 1µM). This BJ486K:Fe(III) complex possesses comparable stability as that determined for salvigenin: Fe(III) under identical experimental conditions (log *K*^{*}_{salvigenin:Fe} = 5.8(2) at pH 2.0; *K*_{D} 1.6 µM).

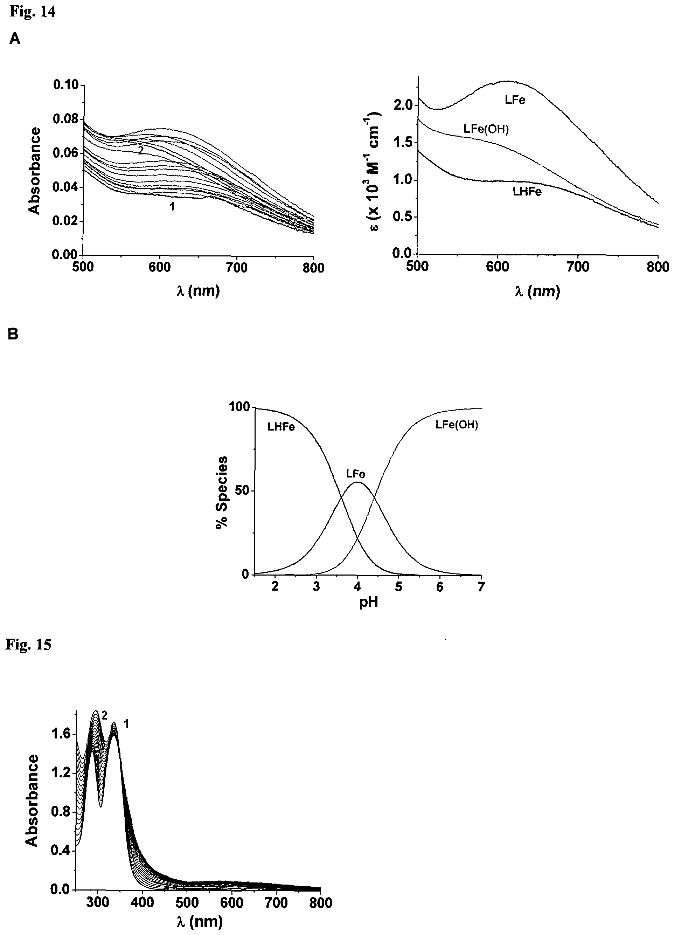

**Figure 14****: Absorption spectrophotometric titration of the ferric complexes of BJ486K vs. pH.** The absorption spectrophotometric titration of the BJ486K:Fe(III) complex versus pH (solvent: CH₃OH/H₂O (80/20 w/w); I = 0.1 M (NEt₄ClO₄); T = 25.0(2) °C; l = 1 cm ; [BJ486K]ₜₒₜ = 4.55 × 10⁻⁵ M ; [Fe(III)]ₜₒₜ = 4.14 × 10⁻⁵ M ; (1) pH = 1.72 ; (2) pH = 6.79.spectra vs. pH (1.72 < pH< 6.79) is shown in **Fig. 13A****.** The diagramme depicted in **Fig. 13B** shows the distribution of the ferric complexes of BJ486K as a function of pH (solvent: CH₃OH/H₂O (80/20 w/w); I = 0.1 M (NEt₄ClO₄); T = 25.0(2) °C; [BJ486K]ₜₒₜ = [Fe(III)]ₜₒₜ = 5 × 10⁻⁵ M). Three protonated species of the monoferric monochelates with the flavone BJ486K were characterized and quantified on the basis of the spectral changes centered on the BJ486K-to-Fe(III) charge transfer absorption. At pH > 7, the fully deprotonated BJ486K:Fe(III) complex (log *K*_{BJ486K:Fe} = 23.8(2)) is the predominant ferric species and the metal cation is monohydroxylated. The 6-hydroxyl unit (cycle A) is also deprotonated. This species undergoes two successive protonations. The first one concerns the hydroxyl ligand coordinated to the metal center (log *K*_{BJ486K:Fe(OH)} = - 4.4(4)). The second one is related to the protonation of the 6-hydroxyl unit (log *K*_{BJ486K:Fe} = 3.6(3)).

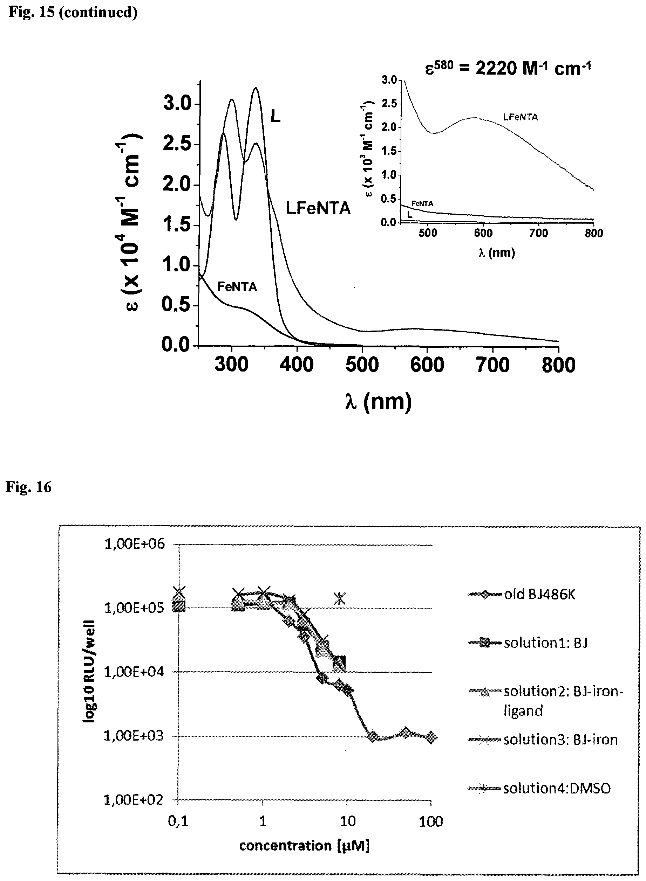

**Figure 15****: Spectrophotometric titration of BJ486K with FeNTA solution at pH = 7.4.** The figure shows the results of an UV-visible absorption spectrophotometric titration of the flavone BJ486K by FeNTA at pH 7.4 (solvent: CH₃OH/H₂O (80/20 by weight); I = 0.1 M (NEt₄ClO₄); T = 25.0(2) °C; pH = 7.4 (hepes 0.1 M); l = 1 cm; (1) [BJ486K]ₜₒₜ = 5.41 x 10⁻⁵ M; (2) [FeNTA]ₜₒₜ/[BJ486K]ₜₒₜ= 2.0). A ternary BJ486K:Fe(III):NTA complex was characterized and quantified (log *β*^{*}_{BJ486K:Fe:NTA} = 10.7(4) at pH 7.4). NTA completes the Fe(III) sphere and prevents hydrolysis of the metal cation, the degradation by autooxidation is not taking the place and the titration can be performed continuously. The BJ486:Fe(III):NTA complex is characterized by the presence of a broad charge-transfer absorption centered at 580 nm with a molar extinction coefficient ε⁵⁸⁰ of 2220 M⁻¹ cm⁻¹. The presence of the NTA binder induces a hypsochromic shift of the charge-transfer band of about 53 nm with respect to the BJ486K:Fe(III) complex.
**Figure 16****: Antiviral activity of the free flavones BJ486K compared to the BJ486K:Fe(III) complex and to the BJ486K:Fe(III):NTA complex.** Huh-7.5 cells were inoculated with F-LucJc1 reporter virus preincubated with the given amount of either BJ486K, BJ486K.Fe(III) complex or BJ486K: Fe(III):NTA complex for 4h at 37°C. 72h post infection, cells were lysed and firefly reporter activity was measured. Mean values of 4 single measurements within one experiment are shown.

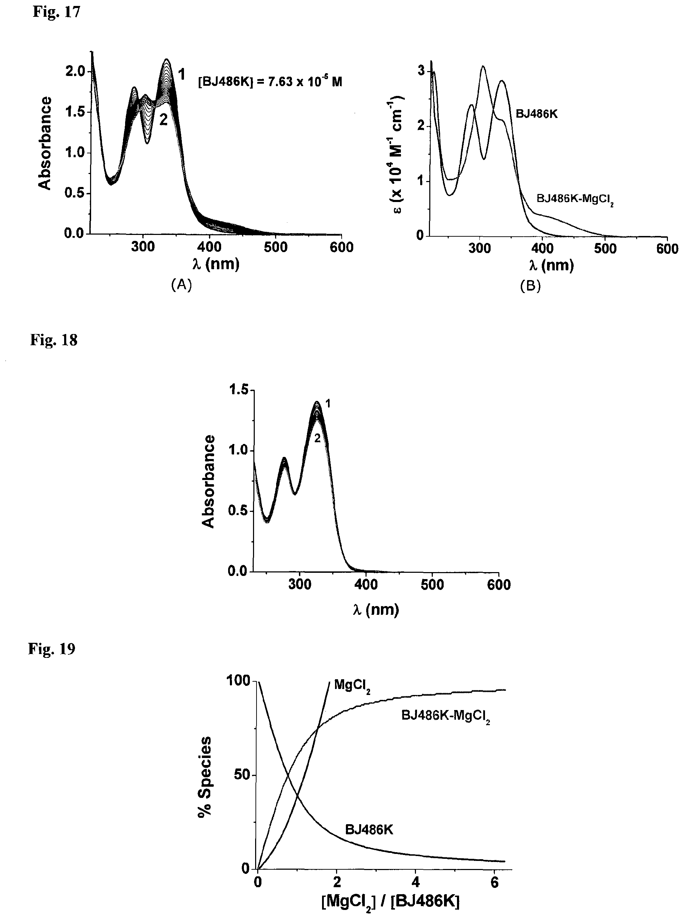

**Figure 17****: (A) UV-visible spectrophotometric titration of BJ486K (newly re-prepared as XM148) by MgCl₂ and (B) absorption electronic spectra of the flavone BJ486K and its magnesium(II) complex BJ486K-MgCl₂.** Solvent: CH₃CN/H₂O (1:1 by volume); *T* = 25.0 °C; *l* = 1 cm; [BJ486K]ₜₒₜ = 7.63 × 10⁻⁵ M; (1) [MgCl₂]ₜₒₜ/[BJ486K]ₜₒₜ = 0; (2) [MgCl_{2]tot}/[BJ486K]ₜₒₜ = 185.8. To ensure solubilisation of both the flavone BJ486K (newly re-prepared as XM148) and MgCl₂, a mixed solvent made of 50% of water and 50% of CH₃CN was used. A stock solution of XM148 (3.0 mg in 10 mL of argon saturated CH₃CN, [XM148]ₜₒₜ = 9.54 × 10⁻³ M) was prepared. 160 µL of the flavone stock solution in CH3CN was diluted in 1 mL and 840 µL of Ar-saturated water and CH₃CN, respectively. The absorption spectrophotometric titration (200 - 800 nm) was carried out by successive addition of aliquots (10 µL) of a stock solution of MgCl₂ freshly prepared in water (337.6 mg in 5 mL, [MgCl₂]ₜₒₜ = 7.09 × 10⁻³ M). The figure shows the corresponding absorption spectral data. Mg(II) complexation was clearly ascertained thanks to bathochromic shift of the band II of the flavone which blends with band I as well as the formation of the new band in the visible region of much lower intensity. The spectroscopic signatures are unambiguous features of BJ486K (XM148) metal coordination processes. Statistical processing of the spectral data allowed calculating the stability of the XM148-MgCl₂ complex (log *K* = 2.4 ± 0.3) which is in good agreement with the very rare physico-chemical data available for flavone/Mg(II) complexes ((a) 2000LXa H Li,H Xie,P Wang,S Wu; New J.Chem.,24,105 (2000)) .
**Figure 18****: (A) UV-visible spectrophotometric titration of XM131 (final precursor B, Scheme 2) by MgCl₂.** Solvent: CH₃CN/H₂O (1:1 by volume); T = 25.0 °C; *l* = 1 cm; [XM131]ₜₒₜ = 7.13 × 10⁻⁵ M; (1) [MgCl₂]ₜₒₜ/[XM131]ₜₒₜ = 0; (2) [MgCl₂]ₜₒₜ/[XM131]ₜₒₜ = 99.4. To get further evidences of Mg(II) complexation by the flavone BJ486K (newly re-prepared as XM148), the final precursor B (noted XM131, the 5-OH protected BJ486K precursor) was titrated by MgCl₂ under identical experimental conditions. 2.8 mg of XM131 was dissolved in 10 mL of Ar-saturated CH₃CN ([XM131]ₜₒₜ = 8.53 × 10⁻⁴ M). An aliquot of 160 µL of the flavone stock solution was diluted in 840 µL + 1 mL of Ar-saturated CH₃CN and water, respectively. Absorption spectra (200 - 800 nm) was recorded after each addition of aliquots (10 µL) of a stock solution of MgCl₂ freshly prepared in water (337.6 mg in 5 mL, [MgCl₂]ₜₒₜ = 7.09 × 10⁻³ M). The figure shows the corresponding absorption spectral data. As anticipated, no absorption variation can be observed even in the presence of high excess of Mg(II) with respect to the final precursor B (XM131). Together with the spectral and thermodynamic data obtained on ferric complexes with BJ486K and salvigenin, these spectroscopic data once again established that the β-hydroxy-ketone bidentate site of BJ486K is the active metal coordination site.
**Figure 19****: Distribution diagram of the Mg(II) complex formation with flavone BJ486K using chloride anion.** Solvent: CH₃CN/H₂O (1:1 by volume); *T* = 25.0 °C. [BJ486K]ₜₒₜ = 0.016 M (equivalent to 40 mg of the flavone in 8 mL of CH₃CN). To simulate the experimental conditions required to perform a one-pot reaction (deprotection/complexation) of XM131 (the final precursor of BJ486K) with MgCl₂, a distribution diagram was calculated using the previously determined stability constant of the BJ486K-MgCl₂ complex (log *K* = 2.4 ± 0.3). The concentration of BJ486K was fixed at 0.016 M, which corresponds to our standard experimental conditions for low scale preparation and the [MgCl₂]/[BJ486K] ratio was varied from 0 to 6.25. The figure shows that a significant excess of MgCl₂ is indeed required for the formation of more than 95% of magnesium(II) complex for [BJ486K]ₜₒₜ = 0.016 M. Using such strategy will certainly lead to formulations containing high excess of MgCl₂, which might interfere with accurate determination of the flavone-MgCl₂ complex content.

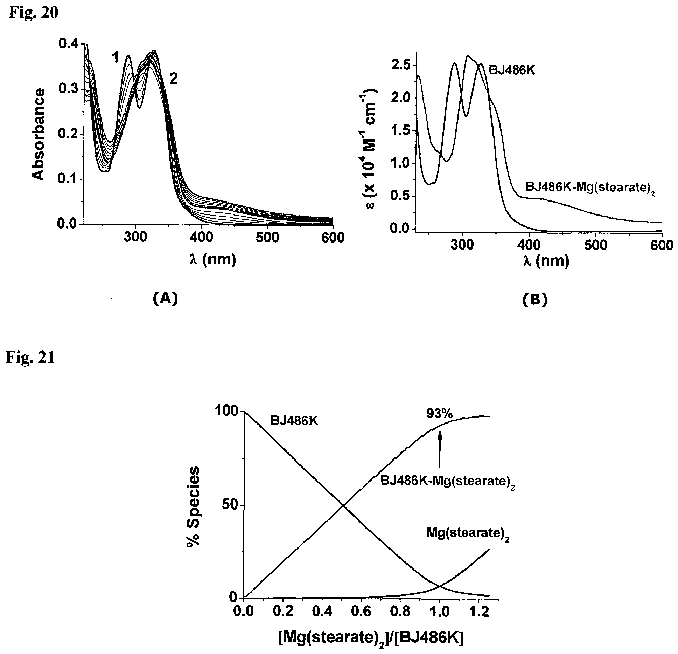

**Figure 20****: (A) UV-visible spectrophotometric titration of BJ486K by Mg(stearate)₂ and (B) absorption electronic spectra of the flavone BJ486K and its magnesium(II) complex BJ486K-Mg(stearate)₂.** Solvent: dioxane; *T* = 25.0 °C; *l* = 1 cm; [BJ486K] = 1.52 × 10⁻⁵ M; (1) [Mg(stearate)₂]/[BJ486K] = 0; (2) [Mg(stearate)₂]/[BJ486K] = 13.26. To overcome the thermodynamic barrier experienced with the MgCl₂ salt, we anticipated that the use of another magnesium(II) salt containing stabilizing auxiliary ligands such as stearic acid might favor complexation by the BJ486K flavone. To ensure adequate solubilisation of both the flavone BJ486K (newly re-prepared as XM148) and Mg(stearate)₂, dioxane solvent was selected. A stock solution of BJ486K (1.6 mg in 5 mL of argon saturated dioxane, [XM148]ₜₒₜ = 1.02 × 10⁻³ M) was prepared. On the other side, 34.3 mg of Mg(stearate)₂ was refluxed in 10 mL of dioxane under argon to ensure appropriate dissolution. An aliquot of 30 µL of the flavone stock solution in dioxane was diluted in 2 mL of Ar-saturated dioxane ([XM148]ₜₒₜ = 1.52 × 10⁻⁵ M). The absorption spectrophotometric titration (200 - 800 nm) was carried out by successive addition of aliquots (5 µL) of a stock solution of Mg(stearate)₂ freshly prepared in dioxane. The figure shows the corresponding absorption spectral data. As previously observed with MgCl₂ and XM148 in CH₃CN/water solvent, Mg(II) complexation was clearly evidenced thanks to bathochromic shift of the band II of the flavone as well as the formation of the less intense absorption in the visible region. These spectroscopic signatures are once again unambiguous features of BJ486K (XM148) Mg(II) complexation. Statistical processing of the spectral data allowed calculating the stability of the XM148-Mg(stearate)₂ complex (log *K* = 4.1 ± 0.6) which is, as anticipated, much higher than that calculated in the case of MgCl₂ salt. These data are therefore strong proofs of our metal coordination strategy and show that Mg(II) can act as an anchor point to introduce, by adequate formulation, the flavone BJ486K and its analogs and a wide variety of auxiliary ligands (solubilizing and/or stabilizing groups, active molecules against enveloped viruses such as cyclosporine, chloroquine...)
**Figure 21****: Distribution diagram of the Mg(II) complex formation with flavone BJ486K using stearate auxiliary anion.** Solvent: dioxane; *T* = 25.0 °C; [BJ486K] = 0.016 M (equivalent to 40 mg of the starting flavone in 8 mL of dioxane).
   To further model the experimental conditions required to perform this formulation reaction using Mg(stearate)₂ reactant, a distribution diagram was calculated using the calculated stability constant of the BJ486K-Mg(stearate)₂ complex (log *K* = 4.1 ± 0.6). The concentration of BJ486K was fixed at 0.016 M, which corresponds to our standard experimental conditions for low scale preparation. The [Mg(stearate)₂]/[BJ486K] ratio was varied from 0 to 1.25. The figure clearly shows that for a stoichiometric ratio [Mg(stearate)₂] : [BJ486K] more than 93% of magnesium(II) complex is formed in solution.

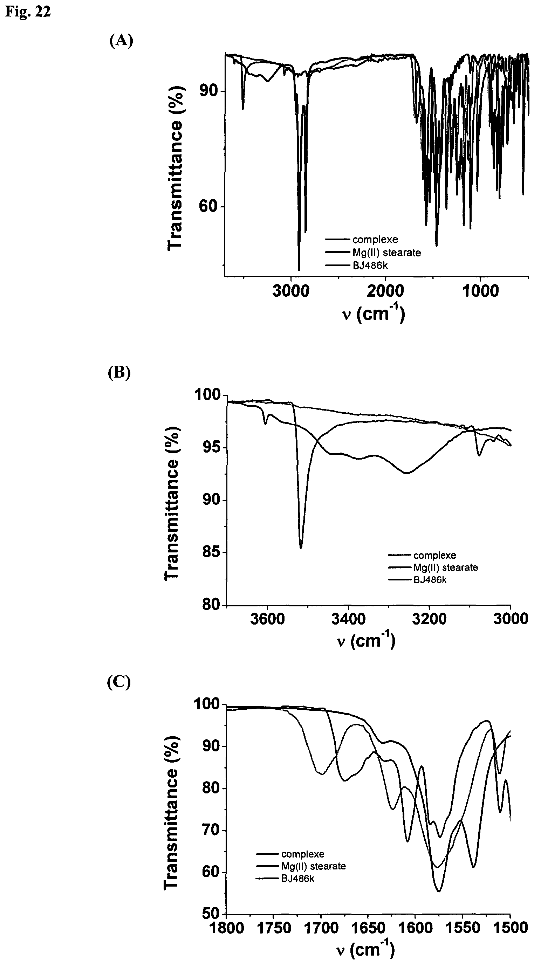

**Figure 22****: IR spectra of Mg(II) stearate, BJ486K and of their complex Mg(II)/stearate/BJ486K.** The figure shows the Infra Red spectrum recorded for the isolated BJ486K-Mg(stearate)₂ complex compared to those of its starting reactants, namely BJ486K and Mg(stearate)₂. These IR spectra show that the hydroxy stretching band appearing at 3157 cm⁻¹ for the free flavone (5-OH) disappears completely once the flavone is complexed to the magnesium(II). Together with the numerous absorption data acquired in this physico-chemical investigation, this feature definitively assess that a chelation process has taken place and that the β-hydroxy-ketone bidentate site of BJ486K is the active metal coordination site. In addition, further evidences of Mg(II) complexation by BJ486K are obtained by comparing the IR bands related to the C=O stretching. For the first C=O band lying at 1675 cm⁻¹ for the free flavone, a Δν(CO) of about 22 cm⁻¹ is measured upon Mg(II) complexation while for the second band centered at 1608 cm⁻¹, a Δν(CO) of about 16 cm⁻¹ is determined. These effects on the C=O stretching bands correlates perfectly with a complexed flavone. More importnantly, no free stearic acid is present in the sample. Stearic acid has a characteristic v(CO) band at about 1700 cm⁻¹, which does not appear in the IR spectrum of the complexed flavone. By contrast, the IR signature of the complexed stearate are still present for the Mg(II)-BJ486K complex clearly indicating that these long chain alkyl-carboxylate auxiliary binders are involved in the Mg(II) coordination.

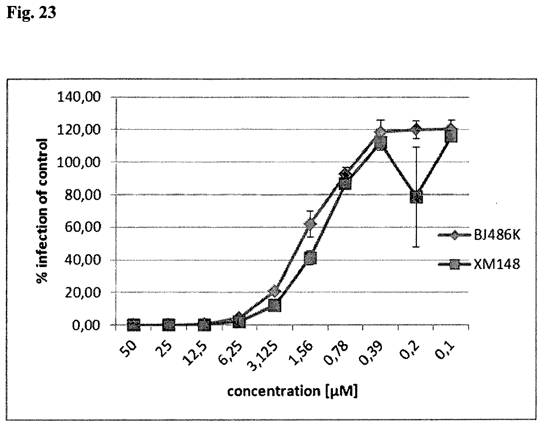

**Figure 23****: Antiviral activity of XM148:** Huh-7.5 cells were incubated for 48h with renilla repoter virus (Jc1R-2a) in the presence of increasing concentrations of XM148 or BJ486K respectively. HCV infection was determined 48h post inoculation using luciferase assays and is expressed as % of cells treated with solvent alone. Results from a single experiment are shown.

The present invention is now further illustrated by the following examples, which are not construed to limit the broad aspects of the invention disclosed herein.

### Examples

### Material and Methods

### Synthesis of Test Compounds

The synthesis of flavone **13** and of its fluorine analogues **14, 15** was executed in a straightforward manner as outlined in Reaction Scheme 1. The commercially available 4,6-dimethoxy-2-hydroxy acetophenone was subjected to Elbs oxidation (Elbs, 1893) using sodium persulfate and aqueous sodium hydroxide to afford the substituted acetophenone **1** in yield between 31 - 64 %. The reaction conditions were applied according to reported procedure but with slight modifications. The yield of the first step is significantly increased upon addition of solid potassium persulfate to the 2-days-old reaction mixture and upon several acidic treatments and heating of the reaction mixture. The generation of the trilithium trianion **2** of the acetophenone **1** was produced by treatment with LiHMDS (Lithium hexamethyldisilylazide). Treatment of trilithium trianion **2** with commercially available benzoyl chloride (**3, 4, 5**), followed by acidification, afforded the diketone intermediates (**6, 7, 8**), which were used without further purification for cyclization. The crude intermediates **6, 7, 8** were heated at 95 - 100 °C in the presence of glacial acetic acid containing 0.5% sulfuric acid for 3.5 h to provide the flavone **9** (in the case of **6**) and 6h to provide flavones **11, 12** (in the case of **7, 8,** respectively). Hydrolysis of the ester function by lithium hydroxide afforded the flavone **10.** Thus, a short and economical synthesis of the flavone **10** was performed in 56% overall yield following a 4 steps-sequence from the acetophenone **1.** In the case of fluorine analogues the cyclization and the cleavage of the ester group was observed in one step. Final demethylation of flavone **10** was achieved in the presence of boron tribromide affording the flavone **13** (herein also referred to as flavone 13, compound 13 or BJ486K; notably, BJ486K is the synthetic counterpart of the structurally identical flavonoid MP03 (also known as ladanein) isolated from plant extracts of *Marrubium peregrinum Lamiaceae*) after hydrolysis by methanol in high 82% yield as well as the demethylation step of flavones **11, 12** to afford **14** and **15** in 93% yield and 27% yield, respectively. Purification of final very pure flavones was performed by low pressure column chromatographic techniques (Sephadex LH20 and CH₂Cl₂/CH₃OH gradients) under nitrogen, and the structures elucidated by NMR and ESI-MS.

A mixture of water (20 mL), dibromomethane(150 mmole) and Adogen 464 (1 mnole) was rigorously stirred and heated to reflux. The air in the system was displaced by Argon. A solution of the appropriate catecholflavones **13-15** (100 mmole) and sodium hydroxide (250 mmole) in water (50 mL) was added at such a rate that the addition was complete after 2 hours. After the addition was complete, the reaction mixture was stirred and refluxed for a further hour. The product **16-18** were then isolated by column chromatographic techniques and identified by NMR and ESI-MS. 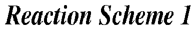 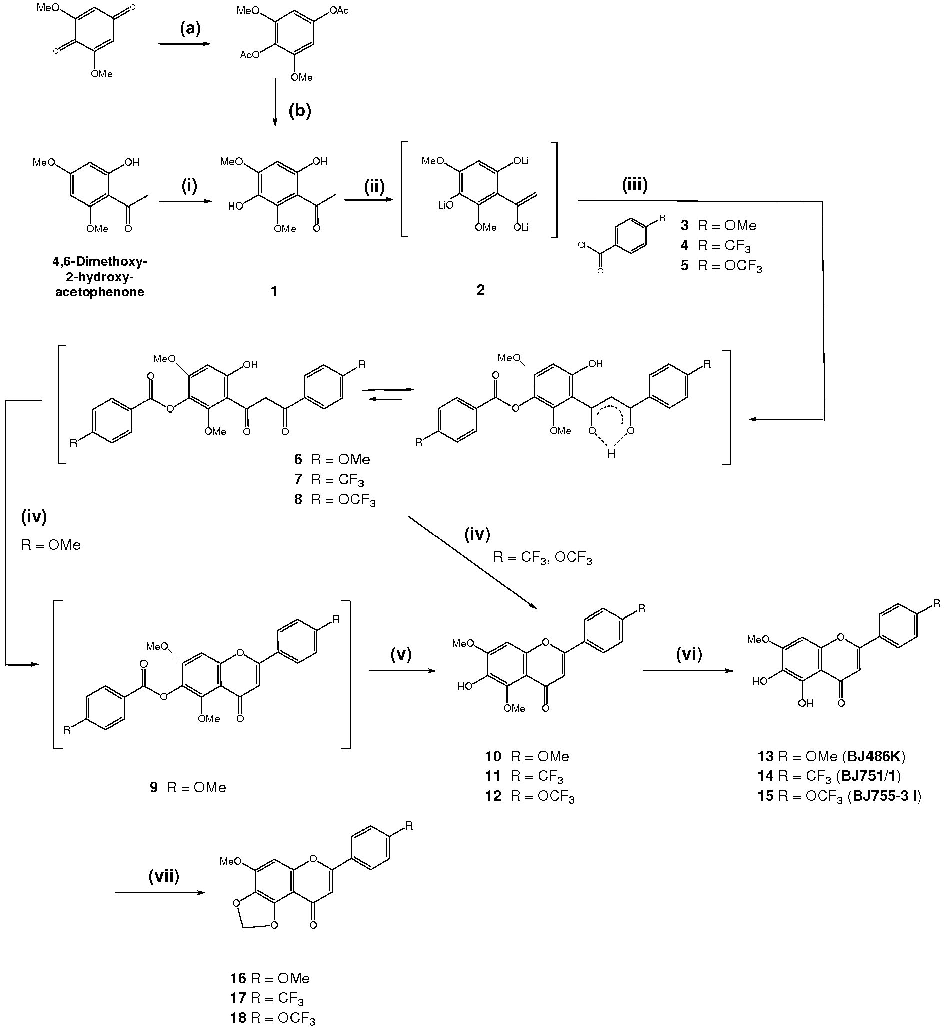 **Reagents and conditions: a**) Zinc, Ac₂O, HCl, 5 min (Yield : 91%); **b**) BF₃-Et₂O, 120°C, 3h (Yield : 53%); **i**) (1) NaOH, K₂S₂O₈, 20 °C, 7 days, (2) Na₂SO₃ (3) HCl; **ii**) LiHMDS, THF, - 78°C, 1 h, - 10 °C, 2h; **iii**) (1) **3a** or **3b** or **3c,** THF, -78 °C (1 h) to 20 °C (overnight), (2) HCl; **iv**) AcOH, H₂SO₄, 95 °C- 100 °C, 3.5 h (for **9**) or 6h (for **11,12**); **v**) THF, LiOH, 20 °C, 2 h; **vi**) (1) BBr₃, CH₂Cl₂, 0 °C, 30 min, (2) MeOH; vii) CH₂Br₂ and Adogen 464 (cat) refluxing in water under Argon, 2h, then add **13-15** and sodium hydroxide (250 mmole) in water.

An improved synthesis of flavones **13-15** was designed in order to obtain the flavone BJ486K in large quantities with a quick and cheap pathway. The final synthesis is depicted in Reaction Scheme 2. Final protection of the β-hydroxy ketone motif of BJ486K was performed by direct complexation with Mg complexes.

The synthesis starts with a reductive acetylation of the 2,6-dimethoxy-1,4-benzoquinone (**1**) in the presence of Zn to yield the diacetate **2** with a quantitative yield. Product **2** undergoes a Fries rearrangement with boron trifluoride etherate in toluene to form the initial precursor of the flavone **3** with 70 % yield after a recrystallization. Both these reactions have been performed with more than 10 g of starting material. Next, the initial precursor is deprotonated in the presence of LiHMDS and undergoes a Baker-Venkataraman reaction in the presence of benzoyl chloride. Without further purification, the heterocycle is formed by heating in acetic acid and sulfuric acid. The final precursor is formed by cleaving the ester bond using LiOH, yielding the flavone **6** with 61 % yield from the initial precursor **3.** No silica gel column chromatography is needed thanks to an acid-base work-up which simplifies the purification. Therefore, the desired product is obtained by a simple recrystallization. Finally, the target molecule **BJ486K** is synthesized with 80 % yield by means of a selective demethylation using magnesium bromide in acetonitrile. To protect the flavone from oxidation, a magnesium complex was designed and prepared in the final step g).

To form the complex, 1 equivalent of BJ486K is dissolved in 1,4-Dioxane to give a yellow solution. Then 0.95 equivalents of magnesium stearate are added. The mixture is refluxed for 1 h. The final solution is orange, which is probably due to the formation of the complex. This observation correlates with the results obtained from the UV-Vis physico-chemical study (apparition of a low intensity band in the visible region). IR spectrum confirms the formation of the complex. The band corresponding to one of the hydroxy groups (3518 cm⁻¹) is no longer present, the bands corresponding to the aromatic rings of the flavone shift slightly and a characteristic band of stearic acid (around 1700 cm⁻¹) is not observed either in the IR spectrum of the complex. 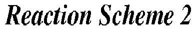 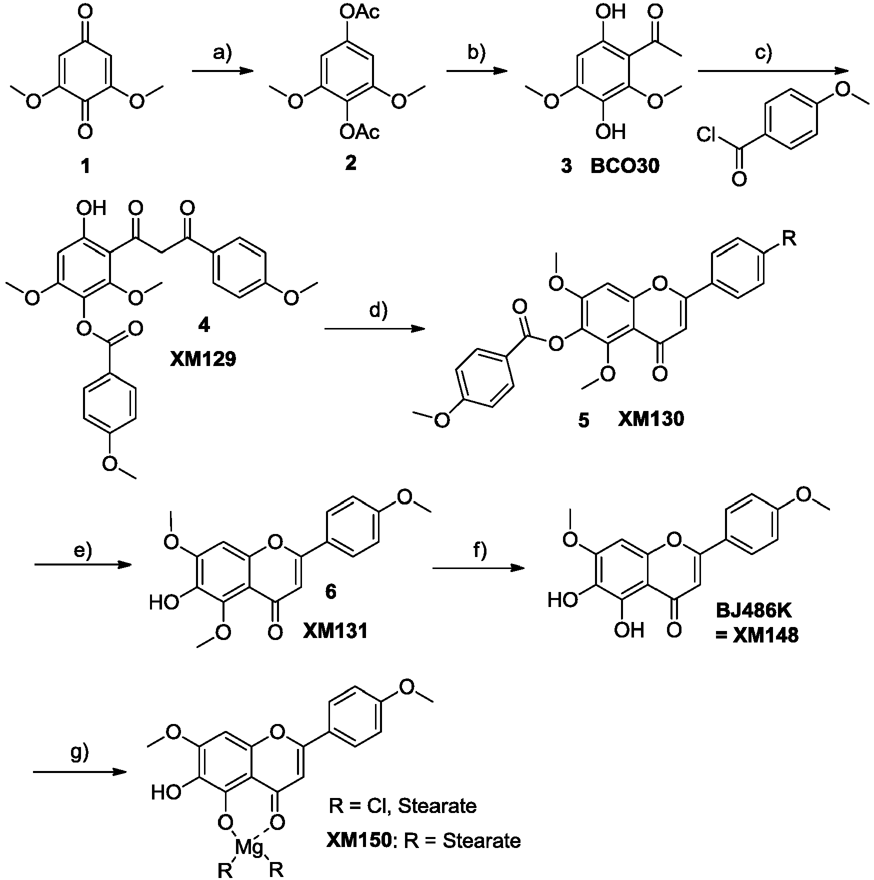 **Scheme 2.** a) Ac₂O, Zn, HCl, 5 min. 0 °C, quant. b) BF₃·Et₂O, PhMe, 110 °C, 18 h, 70 %. c) LiHMDS, THF, -78 °C to RT, 22 h. d) H₂SO₄, AcOH, 100 °C, 3.5 h. e) LiOH, THF, MeOH, 2 h., 61 % from c). f) MgBr₂, MeCN, 80 °C, 3 h., 80 %. g) MgR₂.

**4-(acetyloxy)-3,5-dimethoxyphenyl acetate (2).** A cold (0 °C) solution of concentrated HCl (9 mL, excess) and Ac₂O (50 mL, excess) was added to a mixture of 2,6-dimethoxy-1,4-benzoquinone (2.00 g, 11.89 mmol, 1 equiv) and Zn powder (5.83 g, 89.21 mmol, 7.5 equiv.). The mixture was stirred at 0 °C for 5 min. Et₂O (75 mL) was added and the solid was filtrated. The organic phase was washed with water and brine, dried over magnesium sulfate and concentrated under reduced pressure, adding toluene to coevaporate the acetic acid, to yield a white solid (2.98 g, 11.73 mmol, quantitative yield). No further purification is needed. mp 126 °C; ¹H NMR (CDCl₃; 300 MHz): δ 6.40 (s, 2 H), 3.81 (s, 6 H), 2.34 (s, 3 H), 2.31 (s, 3 H); ¹³C NMR (CDCl₃; 75 MHz): δ 169.4, 168.8, 152.6, 149.1, 126.6, 99.1, 56.4, 21.3, 20.6; IR (neat): 1754, 1615, 1504, 1423, 1179, 1129, 1018, 983, 909, 831, 567 cm⁻¹.

**1-(3,6-dihydroxy-2,4-dimethoxyphenyl)ethan-1-one (3).** BF₃·Et₂O (0.3 mL, 2.36 mmol, 3 equiv.) was added dropwise to a solution of 4-(acetyloxy)-3,5-dimethoxyphenyl acetate (200 mg, 0.79 mmol, 1 equiv.) in toluene (8 mL) and the mixture was heated to 110 °C for 18 h. Then, water (10 mL) and a saturated solution of NH₄Cl (10 mL) are added and the aqueous phase is extracted three times with ethyl acetate. The separated organic layer was washed with water and brine, dried over magnesium sulfate and concentrated under reduced pressure to yield a brown solid. The solid was diluted in CH₂Cl₂, filtered through silica gel, evaporated and recrystallized from CH₂Cl₂/CHex to yield a bright yellow solid (120 mg, 0.55 mmol, 70 % yield). mp 162 °C; ¹H NMR (CDCl₃; 300 MHz): δ 13.20 (s, 1 H), 6.28 (s, 1 H), 5.15 (br. s, 1 H), 3.97 (s, 3 H), 3.94 (s, 3 H), 2.69 (s, 3 H); ¹³C NMR (CDCl₃; 75 MHz): δ 203.4, 158.9, 154.0, 147.4, 131.2, 108.5, 95.5, 60.7, 56.3, 31.6; IR (neat): 3261, 1630, 1594, 1499, 1433, 1078, 1230, 959, 892, 820, 593 cm⁻¹; FAB⁺ MS: 213.2 [M+H]⁺.

**6-Hydroxy-5,7-dimethoxy-2-(4-methoxy-phenyl)-chromen-4-one (6).** A solution of LiHMDS was prepared by adding 20.6 mL of n-BuLi (1.6 M in hexanes, 32.96 mmol, 7 equiv.) to a solution of freshly distilled HMDS (7.2 mL, 34.35 mmol, 7.3 equiv.) in dry THF (20 mL) at 0 °C. The mixture was stirred at that temperature for 30 min and then was added dropwise to a solution of 1-(3,6-dihydroxy-2,4-dimethoxyphenyl)ethan-1-one (1.00 g, 4.71 mmol, 1 equiv.) in dry THF (60 mL) under Ar atmosphere and at -78 °C. The mixture was left to stir for 1 h at that temperature, 2 h at -10 °C and then it was cooled down to -78 °C again. A solution of 4-methoxybenzoyl chloride (1.30 mL, 9.65 mmol, 2.05 equiv.) in dry THF (20 mL) was added and the reaction was stirred for 1 h at that temperature and at room temperature overnight. The solution was poured into a mixture of crushed ice and concentrated HCl, extracted three times with CH₂Cl₂, dried over magnesium sulfate and concentrated under reduced pressure to yield a brown solid. The solid was then dissolved in acetic acid (40 mL), H₂SO₄ (0.5 mL) was added and the reaction was heated at 95 °C for 3.5 h. Then, the solvent was evaporated under reduced pressure, water was added and the aqueous phase was extracted three times with CH₂Cl₂. The organic phase was dried over magnesium sulfate and the solvent was evaporated under reduced pressure to yield a dark brown solid. The solid was then dissolved in THF (50 mL), and a solution of LiOH (700 mg, 29.23 mmol, excess) in MeOH (15 mL) was added. The reaction was stirred at room temperature for 2 h. The solution was poured into water and acetic acid was added until pH 5. The aqueous phase was extracted three times with CH₂Cl₂, then the organic phase was thoroughly washed with a 0.5 M solution of NaOH. The aqueous phase was then acidified to neutral pH with a 1 M solution of HCl and the aqueous phase was extracted three times with CH₂Cl₂. After concentration under reduced pressure and a recrystallization from water/EtOAc, a light brown solid was obtained (0.94 g, 2.86 mmol, 61 % yield). mp 222-223 °C, ¹H NMR (CDCl₃; 300 MHz): δ 7.84 d, *J*=8.9 Hz, 2 H), 7.02 (d, *J*=8.9 Hz, 2 H), 6.84 (s, 1 H), 6.61 (s, 1 H), 5.93 (s, 1 H), 4.04 (s, 3 H), 4.03 (s, 3 H), 3.90 (s, 3 H); ¹³C NMR (CDCl₃; 75 MHz): δ 177.0, 162.1, 161.4, 152.1, 151.9, 144.0, 136.6, 127.6, 124.0, 114.4, 112.3, 106.9, 96.2, 62.6, 56.4, 55.5; IR (neat): 3281, 1602, 1498, 1358, 1249, 1182, 1111, 838, 827, 555 cm⁻¹; FAB⁺ MS: 329.10 [M+H]⁺.

**5,6-Dihydroxy-7-methoxy-2-(4-methoxy-phenyl)-chromen-4-one (BJ486K).** To a solution of 6-Hydroxy-5,7-dimethoxy-2-(4-methoxy-phenyl)-chromen-4-one (152.7 mg, 0.46 mmol, 1 equiv.) in acetonitrile (10 mL) was added MgCl₂ (172.6 mg, 0.92 mmol, 2 equiv.). The reaction mixture was stirred for 3 h. Then a solution of HCl 0.5 M was added and the aqueous phase was extracted with ethyl acetate. The organic phase was washed three times with HCl 0.5 M, water and brine. The separated organic layer was dried over magnesium sulfate and concentrated under reduced pressure to yield a bright yellow solid (116.3 mg, 0.37 mmol, 80 % yield). mp 215 °C dec.; ¹H NMR (DMSO-d6; 300 MHz): δ 12.62 (s, 1 H), 8.73 (s, 1 H), 8.07 (d, *J*= 8.8 Hz, 2 H), 7.13 (d, *J*=8.8 Hz, 2 H), 6.96 (s, 1 H), 6.91 (s, 1 H), 3.94 (s, 3 H), 3.88 (s, 3 H); ¹³C NMR (DMSO-d6; 75 MHz): δ 182.2, 163.3, 162.2, 154.4, 149.6, 146.2, 129.9, 128.2, 123.0, 114.5, 105.1, 103.1, 91.2, 56.3, 55.5; IR (neat): 3518, 1482, 1464, 1359, 1248, 1179, 1108, 1035, 830, 803, 558 cm⁻¹; HRMS (ESI+) : 315.09 [M+H]⁺.

**Magnesium stearate complex of XM148 (XM150).** Magnesium stearate (54.8 mg, 0.093 mmol, 0.94 equiv.) was added to a solution of 5,6-Dihydroxy-7-methoxy-2-(4-methoxy-phenyl)-chromen-4-one (30.9 mg, 0.10 mmol, 1 equiv.) in dioxane (8 mL) at 100 °C. The mixture was heated for one hour and the colour changed from yellow to orange. The solvent was evaporated under reduced pressure and the solid recrystallized in acetonitrile to yield an orange solid. IR (neat): 2917, 2849, 1577, 1456, 1304, 1259, 1178, 1133, 833. Compared **¹H** NMR data of the free flavone and of the magnesium stearate complex in CDCl₃: **¹H NMR (300 MHz, CDCl₃) 5,6-Dihydroxy-7-methoxy-2-(4-methoxy-phenyl)-chromen-4-one:** δ 12.59 (s, 1H), 7.84 (d, *J* = 8.9 Hz, 2H), 7.02 (d, *J* = 8.9 Hz, 2H), 6.59 (s, 2H), 4.00 (s, 3H), 3.89 (s, 3H); **¹H NMR (300 MHz, CDCl₃) Magnesium stearate complex:** δ 7.86 (m), 7.02 (m), 6.56 (m), 4.19 (s), 3.88 (s, 3H), 2.30 (s, 2H), 1.60 (s, 2H), 1.27 (s, 56 H), 0.89 (s, 3H). When both spectra are compared, there is the disparition of the peak corresponding to the hydroxy group in β-position to the carbonyl group. Peaks corresponding to aromatic protons of the flavone have slightly shifted. Peaks corresponding to the stearate appear also slightly shifted (2.30 ppm instead of 2.35 for stearic acid, 1.60 instead of 1.64, 1.27 instead of 1.26 and 0.89 instead of 0.88). Finally, it is interesting to notice that no stearic acid is present since the characteristic peak of the carboxylic group around 11 ppm is not present.

**Magnesium chloride complex of XM148 (XM156).** MgCl₂ (163.3 mg, 0.8 mmol, 5 equiv.) was added to a solution of 5,6-Dihydroxy-7-methoxy-2-(4-methoxy-phenyl)-chromen-4-one (50.5 mg, 0.16 mmol, 1 equiv.) in acetonitrile. The mixture was stirred at 80 °C during an hour. The colour of the mixture changed from yellow to orange. The solvent was evaporated under reduced pressure to yield an orange solid. Compared **¹H** NMR data of the free flavone and of the magnesium chloride complex in **DMF-d7_{:} ¹H NMR (300 MHz, DMF-d7) 5,6-Dihydroxy-7-methoxy-2-(4-methoxy-phenyl)-chromen-4-one:** δ 12.71 (s, 1H), 8.10 (d, *J* = 8.9 Hz, 2H), 7.17 (d, *J* = 8.9 Hz, 2H), 7.00 (s, 1H), 6.84 (s, 1H), 4.00 (s, 3H), 3.93 (s, 3H); **¹H NMR (300 MHz, DMF-d7) Magnesium chloride complex:** δ 8.04 (m, 2H), 7.08 (d, *J*=8.9 Hz, 2H), 6.97 (s, 1H),6.76 (s, 1H), 3.87 (s, 3H), 3.81 (s, 3H). Once again, when the complex is formed, the peak corresponding to the hydroxy group in β-position to the carbonyl group disappears. Also, all other peaks slightly shift due to the complexation.

### Cell Culture

Huh7-Lunet/CD81 cells, a derivative of Huh-7 cells highly permissive for HCV RNA replication and infection (Koutsoudakis et al., J.Virol. 2007;81:588-598), were cultured in Dulbecco's modified Eagle medium (DMEM, Invitrogen) with 10% fetal bovine serum (FCS Gold, PAA, Coelbe, Germany), 1x non-essential amino acids (Invitrogen), 100 µg/ml streptomycin (Invitrogen) and 100 IU/ml penicillin (Invitrogen), 2 mM L-glutamine (Invitrogen) and 750 µg/ml geneticin (Carl Roth, Germany).

### In vitro transcription and electroporation of Huh7-Lunet/CD81 cells

*In vitro* transcripts were generated and transfected using electroporation as described recently (Koutsoudakis et al., J.Virol. 2006;80:5308-5320).

### Luciferase infection assay

Huh7-Lunet/CD81 cells were infected with reporter virus particles essentially as described recently (Koutsoudakis et al., J.Virol. 2006; 80: 5308-5320). Briefly, for inoculation with firefly luciferase reporter virus particles (Luc-Jc1) and *Renilla* reporter viruses (JcR-2a (Reiss et al., Cell host & microbe 2011; 9: 32-45) and GT1-6, see plasmids below), Huh7-Lunet/CD81 cells were seeded at a density of 6×10e4 cells per well of a 12-well plate or 6.6×10e3 cells per well of a 96-well plate 24h prior to inoculation. If not stated otherwise, the virus particles were pretreated with the inhibitors for 1h at 37°C. Cells were inoculated for 4h at 37°C, viral inoculum was replaced by fresh culture fluid and infection was quantified 48h or 72h after virus inoculation using luciferase assays. To this end, cells were washed once with PBS and in the case of the Luc-Jc1 infected cells, lysed directly on the plate with 350 µl of cold lysis buffer (0.1% Triton-X100, 25 mM glycylglycine, 15 mM MgSO₄, 4 mM EGTA and 1 mM dithiothreitol, pH 7.8) and after addition of the luciferase solution (200 µM luciferin, 25 mM glycylglycine, pH8) measured for 20s in a luminometer (Lumat LB9507, Berthold, Freiburg, Germany). Cells infected with the renilla reporter virus were lysed in 200 µl lysis buffer (Promega, Mannheim, Germany) and after addition of the luciferase substrate (1 µM of coelenterazin (P.J.K., Kleinblittersdorf, Germany) in PBS) measured for Is in a luminometer (Lumat LB9507, Berthold, Freiburg, Germany). The indicated IC₅₀ values were determined by using Sigma Plot software (Systat Inc; San Jose; CA).

### Plasmids

The plasmids pFK-Luc-Jc1 and pFK-Jc1 encoding the intragenotypic 2a/2a chimeric virus Jc1 with or without firefly luciferase reporter gene as well as the plasmid pFKi389RLuc2aCore-3'-Jc1 (⁵ referred to as JcR-2a) have been described. Briefly, JcR-2a is a monocistronic reporter virus derived from the Jc1 chimera that expresses a *Renilla* luciferase (RLuc) that is fused N-terminally with the 16 N-terminal residues of the core protein. C-terminally RLuc is connected to the Jc1 open reading frame through the foot-and-mouth disease virus (FMDV) 2A peptide coding region which liberates the reporter from the downstream HCV proteins. This construct was used to create infectious monocistronic RLuc reporter virus genomes for all six major genotypes. Briefly, chimeric HCV open reading frames encoding core, E1, E2, p7 and NS2 of the GT1a isolate H77C, the GT1b isolate J4, the GT2b isolate J8, the GT3a isolate S52, the GT4a isolate ED43, the GT5a isolate SA13, or the GT6a isolate HK6a, respectively, followed by JFH1-derived NS3 to NS5B genes were cloned downstream of the R2a-reportergene cassette. The resulting novel monocistronic *Renilla* luciferase reportervirus genomes were designated H77c/1a/R2a, J4/1b/R2a, J8/2b/R2a, S52/3a/R2a, ED43/4a/R2a, SA13/5a/R2a, HK6a/6a/R2a.

### Infection assays with HIV and vesicular stomatitis virus

*Env*-deleted HIV-1-GFP reporter viruses, which had been pseudotyped with the R5 envelope from YU-2, were incubated with various concentrations of BJ486K or its naturally occurring counterpart MP03 extracted and isolated from plant extracts of *Marrubium peregrinum Lamiaceae.* Briefly, TZM-bl cells were challenged with these viruses overnight, washed and subsequently cultivated under standard conditions. Three days post-infection the percentage of infected, GFP-positive cells was quantified by flow cytometry. Values obtained for solvent-treated controls were set to 100%. Shown are arithmetic means ± SD (n=3) from one of three independent experiments. IC₅₀ values were determined by using Prism software (GraphPad, San Diego, CA).

Replication competent GFP-tagged vesicular stomatitis virus (VSV-GFP) was incubated with various concentrations of BJ486K for 1h at 37°C prior to infection of Vero-CH, A549 or Huh-7.5 cells, respectively. Virus titers were analysed using limiting dilution assays. In brief, cells were inoculated with serially diluted virus preparations with six replicate wells of a 96-well plate per dilution. Cells were fixed with 3% paraformaldehyde 48h to 72h after infection and in the case of CoxV and AdV5 infection stained with coomassie solution (0.25% Coomassie brilliant blue R-250, 45% methanol, 10% acetic acid) to identify infected wells. In case of VSV infections, we used a recombinant vesicular stomatitis virus expressing enhanced green fluorescent protein designated VSV*M_{Q}. This virus carries four attenuating mutations in the M protein, and expresses GFP from an additional transcriptional unit located between G and L. VSV*M_{Q} infected cells were identified by detection of GFP-autofluorescence using a Olympus IX-81 microscope. Virus titers (50% tissue culture infective dose [TCID₅₀/ml]) were calculated based on the method of Spearman and Kärber.

### Freeze and thaw lysis of HCV-transfected cells

Huh7-Lunet/CD81 cells were transfected with Jc1 RNA and seeded on a 6-well plate. Compounds were added 4h later. At 48h after transfection, cell culture supernatant was harvested and used for measuring the extracellular core amount. Cells were washed once with PBS, scraped and centrifuged for 5 min at 1000×g. Cell pellets were resuspended in 500 µl fresh culture fluid and subjected to three cycles of freeze and thaw using liquid nitrogen and a 37°C thermo block. Samples were than centrifuged at 10,000xg for 10 min to remove cell debris and the core amount was measured (intracellular core protein) using the ARCHITECT HCV Core AG test (Abbott, Wiesbaden, Germany) according to the instructions of the manufacturer.

### Cytotoxicity assay

Cytotoxicity was measured using the CytoTox-Glo cytotoxicity assay (Promega, Mannheim, Germany) as described by the manufacturer employing a plate luminometer Centro XS LB960 (Berthold, Freiburg, Germany) according to the manufacturer's instructions.

### Determination of antiviral activity against early steps of HCV cell entry

JcR-2a virus particles were incubated with Huh7-Lunet/CD81 cells seeded as described above for 1h at 4°C to synchronize cell entry. Various inhibitors including 50 µg/ml heparin (Sigma-Aldrich), CD81-specific antibody JS-81 (2 µg/ml; Becton Dickinson), ITX 5061 (10 µM) or BJ486K (20 µM), were added during virus inoculation (protocol 1) or at indicated time points after virus inoculation (protocol 2-8). After 4h inhibitor treatment, medium was changed to fresh culture fluid without inhibitors and the cells were lysed 48h after inoculation as described above. Efficiency of infection was determined by luciferase assay using a tube luminometer (Lumat LB9507, Berthold, Freiburg, Germany).

### Immunohistochemical staining and virus titration

Virus titers were measured using the limiting dilution assay as described recently (Steinmann et al., J.Virol. 2008; 82: 7034-7046). Virus titers (50% tissue culture infective dose [TCID₅₀/ml]) were calculated based on the method of Spearman and Kärber (Spearman C., British Journal of Psychology 1908; 2: 227-242; Kärber G., Archiv für experimentelle Pathologie und Pharmakologie 1931; 162: 480-487).

### Infection of primary human hepatocytes

Normal-appearing liver tissue from patients seronegative for HCV, hepatitis B virus, and human immunodeficiency virus (HIV) was obtained from adult patients undergoing partial hepatectomy for the therapy of metastases. Experimental procedures were carried out in accordance with French Laws and Regulations. Hepatocytes were isolated by a two-step perfusion technique and maintained in primary culture as described previously (Podevin et al., Gastroenterology 2010; 139: 1355-64). Cells were inoculated with Con1/C3-HCV (Pietschmann et al., Proc. Natl. Acad. Sci. U.S.A 2006; 103: 7408-7413) grown in Huh-7.5.1 cells at a multiplicity of infection of 0.1 in the presence of dimethylsulfoxide as carrier control, or else increasing concentrations of BJ486K. Three days later, infectivity titers in culture supernatants were determined by focus-formation assay as previously described by (Pene et al., Journal of Viral Hepatitis 2009;16:705-15).

### BJ486K inhibits HCV cell entry but not RNA replication or virus production

To characterize the antiviral effect of BJ486K, the impact of BJ486K on the steps of HCV RNA translation and replication was assessed. To this end, Huh7-Lunet/CD81 cells were transfected with a subgenomic JFH1 luciferase replicon and RNA replication was monitored 72h later by luciferase assays. As control we incubated the cells with increasing doses of the NS3-4A protease inhibitor boceprevir and the polymerase inhibitor 2'-C-methyladenosine (2'CMA). As shown in Figure 2A, BJ486K affected neither RNA translation nor replication of HCV RNA as luciferase activity was not changed up to a dose of 10µM.

To determine whether BJ486K affects virus assembly or release we transfected Huh7-Lunet/CD81 cells with Jc1 RNA and quantified intra- and extracellular levels of core protein 48h after transfection (Figure 2B). As expected, both quinidine and the grapefruit flavonoid naringenin, two assembly inhibitors recently identified using the JFH1-based infection system, selectively reduced extracellular levels of core protein by more than 100- or ca. 5-fold, respectively, thus confirming their interference with HCV virus production. In contrast, BJ486K reduced neither intracellular nor extracellular core protein levels up to a dose of 20 µM, which is 10-fold higher than the determined IC50 value (Figure 1). Collectively these data indicate that different flavonoids can prevent HCV propagation by distinct mechanisms and that BJ486K does not impede HCV RNA translation, RNA replication and virus production. In agreement with this conclusion, addition of BJ486K only interfered with HCV infection when the drug was present during virus inoculation, whereas addition of the compound before or after virus inoculation did not preclude productive cell entry and subsequent RNA replication (Figure 2C).

### BJ486K interferes with a post-attachment step of HCV cell entry

To resolve which step of HCV cell entry is inhibited by BJ486K, we assessed its antiviral activity when administered at different time points during the early phase of infection. To this end, Huh7-Lunet/CD81 cells were inoculated for one hour at 4°C with a reporter virus prepared in the absence of drugs. At this temperature, virus particles bind to the cell surface but do not efficiently enter, thus permitting a rather synchronous infection when the inoculum is removed and cells are shifted to 37°C. A selection of HCV entry inhibitors which prevent infection by different mechanisms or BJ486K were added during inoculation, directly afterwards or at different intervals shortly thereafter. To attain maximal sensitivity we used a novel monocistronic renilla luciferase reporter virus based on the intragenotypic genotype 2a chimera Jc1 which is designated JcR-2a and that was described recently (Reiss et al., Cell host & microbe 2011; 9: 32-45) (Figure 3). As expected, heparin which is known to impede HCV cell attachment only interfered with HCV infection when present already during inoculation (Figure 3). In contrast, BJ486K like CD81-specific antibodies, potently inhibited entry not only when present during inoculation but also when added directly after virus binding (>90% inhibition). In fact, administration of the drug 20 minutes post virus attachment still inhibited ca. 80% of HCV infection, clearly indicating that BJ486K blocks a post-binding step of the HCV entry pathway. The kinetics of BJ486K-mediated blockade of HCV cell entry were comparable to CD81-specific antibodies and also to ITX5061, a clinical stage compound that is thought to interfere with HCV infection through inhibiting viral interaction with SR-BI.

**BJ486K is not affected by viral resistance against a SR-BI-targeting entry inhibitor, inhibits HCV of all major genotypes and impedes infection of primary human hepatocytes.** Syder et al. recently reported that a specific mutation within the HCV envelope protein E2 (G451R) that is known to reduce viral dependency on SR-BI for cell entry (J.Hepatol. 2011; 54: 48-55) at the same time reduces the susceptibility of HCV towards inhibition by ITX5061²⁸. These data indicate that decrease of SR-BI receptor dependence by HCV is a possible viral escape mechanism from inhibition by SR-BI-targeting antivirals. Therefore, to explore whether this viral resistance mechanism also compromises the antiviral activity of BJ486K we compared the antiviral activity of the two compounds against wild type and the mutant virus carrying the G451R exchange within E2. In line with the report by Syder et al., the aforementioned mutation rendered HCV resistant to inhibition by ITX5061 (Figure 4A). In contrast, the antiviral activity of BJ486K was not changed by this mutation (Figure 4B), indicating that reduced SR-BI dependence does not confer resistance towards entry inhibition by BJ486K.

Efficacy of current HCV treatment is substantially influenced by viral genotype. More specifically, IFN-based therapy regimens attain sustained viral response rates of ca. 80% in patients infected with genotype 2 and 3 viruses whereas response rates among patients chronically infected with genotype 1 reach only about 50%³⁰. Moreover, directly targeted antivirals in late stage clinical development vary in efficacy depending on the viral genotype. Therefore, we determined the antiviral activity of BJ486K towards HCV particles from different genotypes. To be able to resolve different response rates among these viruses with highest resolution, we created a set of novel reporter virus constructs based on the JcR-2a backbone (Figure 3). Importantly, antiviral activity of BJ486K against cell culture derived particles from genotype 1a, 1b, 2b, 3a, 4a, 5a and 6a was comparable and showed an IC50 below 4.2 µM indicating that BJ486K inhibits HCV cell entry independently of viral genotype or subtype (Figure 4C and Table 1).

**Table 1. Antiviral activity of BJ486K across all major HCV genotypes**

| **HCV Isolate** | **IC₅₀ [µM]** | **IC₉₀ [µM]** |
|---|---|---|
| H77c (GT1a) | 2,46 | 11,64 |
| J4 (GT1b) | 2,35 | 9,29 |
| J8 (GT2b) | 4,14 | 8,42 |
| S52 (GT3a) | 2,89 | 10,65 |
| ED43 (GT4a) | 0,74 | 1,78 |
| SA13 (GT5a) | 1,39 | 5,33 |
| HK6a (GT6a) | 0,75 | 2,39 |

Although human hepatocellular carcinoma derived cell lines sustain the entire HCV replication cycle and are a well-accepted model for HCV infection studies, these cells nevertheless functionally differ from primary human hepatocytes. Therefore, to confirm the antiviral activity of BJ486K in a more natural system, primary human hepatocytes were inoculated in the presence or absence of BJ486K with a cell culture derived genotype 1b virus (Con1/C3; ²⁴). As shown in Figure 4D, Con1/C3 infection of primary human hepatocytes was inhibited by BJ486K in a dose-dependent fashion with an IC50 value of ca. 10µM.

**Bioavailability of BJ486K.** Mice (24 in total) were treated either orally or intraveniously with a single dose of 0.25 mg/kg of the inhibitor (solved in saline/10% cremophor EL). At different time points after inhibitor administration (5 min, 15 min, 30 min, 60 min, 120 min or 180 min) two mice from each group were sacrified and the blood was centrifuged to obtain the plasma, which was stored at -80°C until further examination. 400 µl of each plasma sample was mixed with equal amount of a 2% trifluoroacetic acid and loaded on a SPE Strata C18 column (equilibrated with 1 ml methanol and 1 ml 1% trifluoroacetic acid) for solid phase extraction of the plasma. After several washing steps (1% trifluoroacetic acid and water), the samples were eluted with acetonitrile and dried. The remaining sample was resolved in 25 µl acetonitrile:water (ratio 1:1). Samples were further analyzed with a LC-MS/MS (triple quadrupole mass spectrometer LCMS 8030 Shimadzu), which was calibrated at the day of measurement. Plasma drug concentrations versus time curves were plotted for each route. Bioavailability was calculated by dividing the area under curve (AUC) for oral route by the AUC for intravenous route. The results are shown in Figure 10.

**BJ486K exerts synergistic antiviral activity with Cyclosporine A (CsA) and prevents productive infection of differentiated human hepatoma cells.** Next, we investigated if combination of CsA, an inhibitor of HCV RNA-replication *in vitro* and *in vivo* and also an immunosuppressive drug used in the HCV post-transplant setting, with BJ486K would increase antiviral potency. To this end, we challenged Huh7-Lunet/CD81 cells differentiated by DMSO-treatment with the highly infectious Jc1 chimera in the presence of CsA, BJ486K or a combination of both drugs (Figure 5). In this quiescent, growth arrested cell culture we monitored virus production for 12 days after inoculation to find out if drug treatment had completely eliminated virus infection. The applied dose of CsA was not sufficient to prevent infection of the cells and resulted in only about 5 to10-fold lower levels of infectious virus 12 days after inoculation (Figure 5B). In contrast, BJ486K reduced infection almost down to background levels (Figure 5C). Remarkably, combination of both drugs completely eliminated infection as is evident from absence of detectable infectious virus at days 6, 9 and 12 post inoculation (Figure 5D). To obtain a more quantitative measure for the combined antiviral effect of BJ486K and CsA, we inoculated naive Huh7-Lunet/CD81 cells with JcR-2a virus together with varying doses of both drugs (Figure 6A). Importantly, addition of 1µM of BJ486K decreased the IC50 of CsA by more than 10-fold (left panel) and vice versa, as little as 100 ng/mL of CsA decreased the IC50 of BJ486K by ca. 3-fold to ca. 400 nM. Therefore, based on the combination index calculation described by Zhao et al.(Clin Cancer Res 2004; 10: 7994-8004) BJ486K and CsA synergistically inhibited HCV infection (Figure 6B; Table 2). Of note, first pharmacokinetic data of BJ486K administered to mice indicate bioavailability of the drug and peak plasma levels of 329 nM after a single oral dose of 0.25 mg/kg (Figure 10). Collectively, these results indicate that combination of these inhibitors dramatically increases antiviral potency which is sufficient to prevent infection of cultured cells with a high dose of infectious HCV.

**Table 2. Combined effect of BJ486K and CsA**

| **inhibitors** | **IC₉₀** | **CI** |
|---|---|---|
| BJ486K | 4,37 µM | |
| CsA | >1000 ng/ml | |
| BJ486K + 100 ng/ml CsA | 3,03 µM | 0,793 |
| BJ486K + 200 ng/ml CsA | 2,8 µM | 0,840 |
| BJ486K + 300 ng/ml CsA | 2,5 µM | 0,872 |

As new drug combination with BJ486K, the antimalarial chloroquine (CQ) known as an HCV entry inhibitor (Ashfaq, U.A. et al., Virol J. 2011, 8:163) was tested in drug combination with the flavone BJ486K and analogs, in conditions similar to the one performed with BJ486K + CsA. Chloroquine alone has a dose-dependent anti-HCV effect (with no cytotoxicity detectable at the used concentrations, Fig 11 A). An additive effect (depicted in % change over expected value (no mountain, no valley) was observed with the flavone BJ486K and the known lysosomotropic agent CQ (Figure 11 B). This effect was seen in all repeat experiments with no significant difference.

### Metal complexes of BJ486K

### General remarks

The physico-chemical properties of BJK486K and its ferric complexes were examined in a mixture methanol/water (80/20 by weight). Distilled water was further purified by passing through a mixed bed of ion-exchanger (Bioblock Scientific R3-83002, M3-83006) and activated carbon (Bioblock Scientific ORC-83005). Both, distilled water and spectroscopic grade methanol (Merck, Uvasol®, p.a.) were deoxygenated using CO₂- and O₂-free argon prior to use (Sigma Oxiclear cartridge). All the stock solutions were prepared by weighing solid products using an AG 245 Mettler Toledo analytical balance (precision 0.01 mg). The complete dissolution of the ligands was obtained with the help of ultrasonic bath. The ionic strength was adjusted to 0.1 M with tetraethylammonium perchlorate (NEt₄ClO₄.H₂O, Fluka, puriss.). Iron(III) perchlorate stock solutions (Fluka) were freshly prepared in water at acidic pH (pH < 1.5) immediately before use and their concentrations were ascertained by UV-visible absorption spectrophotometry (ε³³⁸= 2,115 x 10⁴ M⁻¹ cm⁻¹ et ε²⁶⁸= 1,845 x 10⁴ M⁻¹ cm⁻¹; R. Bastian, R. Weberling, F. Palilla, "Determination of Iron by Ultraviolet Spectrophotometry" Anal. Chem., 1956, 28, 459-462.). The glassware used was rinsed after each experiment with a hydrochloric acid solution to remove all traces of iron.

The free hydrogen ion concentrations were measured with a combined glass electrode (Metrohm 6.0234.500, Long Life) filled with 0.1 M NaCl (Fluka, p.a.) in MeOH/H₂O (80/20 by weight). Potential differences were given by an automatic titrator system DMS 716 Titrino (Metrohm). The combined glass electrode was calibrated as a hydrogen ion concentration probe by titrating known amounts of perchloric acid (∼ 10⁻¹ M from HClO₄, Fluka, ∼ 70% in water) with CO₂-free tetraethylammonium hydroxide solution (∼ 10⁻¹ M from Et₄NOH, Merck, ∼ 40% in water) in MeOH/H₂O solvent (80/20 by weight). The HClO₄ and NEt₄OH solutions were freshly prepared just before use and titrated with sodium tetraborate decahydrate (B₄Na₂O₇.10H₂O, Fluka, puriss, p.a.) and potassium hydrogen phthalate (C₈H₅KO₃, Fluka, puriss, p.a.), respectively, using methyl orange and with phenolphthalein (Prolabo, purum) as the indicators. The titration cell was thermostated at 25.0(2) °C by the flow of a Lauda E200 thermostat. A stream of argon, pre-saturated with water vapor, was passed over the surface of the solution. The Glee program (P. Gans, B. O'Sullivan "GLEE, a new computer program for glass electrode calibration" Talanta 2000, 51, 33) was applied for the glass electrode calibration (determination of the standard electrode potential E₀/mV and of the slope of the electrode/mV.pH⁻¹) and to check carbonate levels of the NaOH solutions used (< 5% within all the solutions tested).

### Fe(III) coordination properties of BJ486K

### Determination of BJ486K/Fe(III) complex stoichiometry - Job's plot

For the characterization of the BJ486K:Fe(III) complexes (method of continuous variations), stock solutions of BJ486K and iron(III) were freshly prepared under acidic conditions ([HClO₄]ₜₒₜ = 10⁻² M; pH ∼ 2.0) and were diluted to obtain the same concentration for both solutions at 4.94 × 10⁻⁵ M. The ionic strength was kept constant at 0.1 M with tetraethylammonium perchlorate (Et₄NClO₄, Fluka, puriss.). UV-Vis absorption spectra of mixtures with different [BJ486K]ₜₒₜ/([BJ486K]ₜₒₜ +[Fe(III)]ₜₒₜ) ratios were measured in a 1 cm Hellma optical cell with a Cary 300 (Varian) spectrophotometer. The Job's plot was processed with Origin program. The results are shown in Figure 12. The BJ486K:**Fe(III)** complex predominantly has a 1:1 stoichiometry with the metal binding site being the β-hydroxy-ketone unit as evidenced by a comparative study with salvigenin, a 6-methylated BJ486K derivative.

### Spectrophotometric titration of BJ486K with Fe(III) solution at pH 2.0

Stock solution of BJ486K was prepared in CH₃OH/H₂O (80/20 by weight) and then freshly diluted with HClO₄ (10⁻² M) to obtain a ligand concentration of ∼ 5.92 × 10⁻⁵ M. The ionic strength was kept constant at 0.1 M with tetraethylammonium perchlorate (Et₄NClO₄, Fluka, puriss.). The spectrophotometric titrations of BJ486K by Fe(III) were thus carried out on solutions at pH ∼ 2 (10⁻² M HClO₄). Microvolumes of a concentrated solution of Fe(III) (2.4 × 10⁻⁴ M) were added to an aliquot of 0.5 mL of the ligand solution in a 1 cm path length optical Hellma cell (the [Fe(III)]ₜₒₜ/[BJ486K]ₜₒₜ ratio varied from 0 to 1.60). Special care was taken to ensure that complete equilibration was attained. The corresponding UV-Vis spectra were recorded from 230 nm to 800 nm on a Cary 300 (Varian) spectrophotometer maintained at 25.0(2) °C by the flow of a Lauda E200 thermostat. The results are shown in Figure 13 and confirm that flavone BJ486K firmly binds Fe(III) through its β-hydroxy-ketone chelating unit.

### Absorption spectrophotometric titration of the ferric complexes of BJ486K vs. pH

Spectrophotometric titration of the Fe(III) complexes with flavone BJ486K as a function of pH was performed. Due to Fe(III)-catalyzed oxidation of BJ486K under neutral conditions, a specific protocol was adopted. A stock solution of BJ486K was freshly prepared by quantitative dissolution of a solid sample in CH₃OH/H₂O (80/20 by weight), which was further diluted to get a ligand concentration of 4.55 × 10⁻⁵ M. The ionic strength was adjusted to 0.1 M with NEt₄ClO₄ (Fluka, puriss). For each pH measurement, an aliquot of 2 mL of the solution was introduced into a 1 cm Hellma quartz optical cell and 37.0 µL of the Fe(III) stock solution (2.24 × 10⁻³ M) was added. After each addition of base (Et₄NOH) or acid (HClO₄), the solution was mixed and its corresponding pH value was measured using a combined glass electrode (Metrohm 6.0234.500, Long Life) and an automatic titrator system 716 DMS (Metrohm). Special care was taken to ensure that complexation equilibration was attained. The absorption spectra *vs*. pH (1.72 < pH< 6.79) were thus recorded using a Cary 300 (Varian) spectrophotometer. The results are shown in **Figure 13****.** Three protonated species of the monoferric monochelates with the flavone BJ486K exist.

### Fe(III) coordination properties of BJ486K in the presence of an exogenous NTA ligand at pH 7.4

With respect to the biological tests of the antiviral activity of BJ486K performed at pH 7.5-8, it was of importance to find appropriate conditions to study iron(III) complexation by BJ486K under comparable conditions. Exogenous chelators such as citrates or nitrilotriacetic acid (NTA) have been usually used to ensure iron solubility at pH 7.4 ((a) G. W. Bates, C. Billups, P. Saltman "The Kinetics and Mechanism of Iron(III) Exchange between Chelates and Transferrin. I. The Complexes of Citrate and Nitrilotriacetatic Acid" J. Biol. Chem. 1967, 242, 2810-2815. b) Y. Li, W. R. Harris, A. Maxwell, R. T. A. MacGillivray, T. Brown " Kinetic Studies on the Removal of Iron and Aluminum from Recombinant and Site-Directed Mutant N-Lobe Half Transferrins" Biochemistry 1998, 37, 14157-14166. c) J. M. El Hage Chahine, R. Pakdaman " Transferrin, a Mechanism for the Holoprotein Formation from Nitrilotriacetato-Fe(III)-Transferrin Mixed Complex" J. Chim. Phys. 1996, 93, 283-299. d) M. Gabricevic, D. S. Anderson, T. A. Mietzner, A. L. Crumbliss " Kinetics and Mechanism of Iron(III)-Nitrilotriacetate Complex Reactions with Phosphate and Acetohydroxamic Acid" Biochemistry 2004, 43, 5811-5819). We therefore used NTA in combination with BJ486K to characterize and quantify the corresponding ferric complexes. A stock solution of BJ486K (5.41 × 10⁻⁵ M) was freshly prepared in hepes aqueous buffer at pH 7.4 (0.1 M in CH₃OH/H₂O, 80/20 by weight) to obtain a ligand solution about. The ionic strength was adjusted to 0.1 M with tetraethylammonium perchlorate (Et₄NClO₄, Fluka, puriss.). The absorption spectrophotometric titration of BJ486K by FeNTA was carried out in a Hellma quartz optical cell (*l* = 1 cm). Microvolumes of a concentrated solution of FeNTA stock solution (2.41 × 10⁻³ M) were added to an aliquot of 2.0 mL of the ligand solution in a 1 cm path length optical Hellma cell (the [FeNTA]ₜₒₜ/[BJ486K]ₜₒₜ ratio varied from 0 to 2.0). After each addition the solution was mixed and special care was taken to ensure that complete equilibration was reached. The corresponding UV-Visible spectra were recorded on a Cary 300 (Varian) spectrophotometer. The results are shown in Figure 15. A ternary BJ486K:Fe(III):NTA complex is formed which is soluble under physiological conditions at pH 7.4.

### Antiviral activity of the BJ486K:Fe(III) and BJ486K:Fe(III):NTA complex

The iron(III):BJ486K(:NTA) complex solutions were prepared as follows. Stock solutions were prepared and 200 µL of each solution was used:
1.) 400 µM BJ486K solution in 10% DMSO - 90% H₂O
2.) 400 µM BJ486K : Iron : ligand solution 10% DMSO - 90% H₂O
3.) 400 µM BJ486K : Iron solution 10% DMSO - 90% H₂O
4.) 10% DMSO - 90% -H₂O

All final dilutions with medium DMEM high glucose were carried out immediately before addition of the solutions to the virus particles. Additionally, before use, the final solutions were checked for final concentration spectrophotometrically:
1.) 4 mM BJ486K solution in 100% DMSO
2.) Iron : ligand solution* in 100% H₂O, pH 7.4
3.) Iron solution* in 100% H₂O (freshly prepared on the day of testing)
* Stock solution were filtrated for sterilization

Specifically, the following stock solutions were prepared on the day of testing the compounds on their antiviral activity in a corresponding assay described above.
1.) 10% DMSO - 90 % H₂O
2.) Preparation of 400 µM BJ486K/iron/ligand complex solutions in 10% DMSO - 90% H₂O from stock solutions
3.) Dilution of 4.0 mM stock solution BJ486K in 100% DMSO to 400 µM solution in 10% DMSO - 90% H₂O

Final 1/10 dilution of 400 µM solutions in 10% DMSO - 90% H₂O also employed virus particles and DMEM. The results are shown in Figure 16. BJ486K:Fe(III) complexes, in the presence and absence of NTA, inhibit HCV cell entry with the same potency as the uncomplexed BJ486K.

### The physicochemistry of BJ486K and analogues, and of Magnesium complexes (including MgCl₂, MgBr₂, Mg stearate)

To prevent oxidation of the BJ486K flavone either in solution (i.e. we previously demonstrated - see above in the introduction - that base-catalyzed oxidation of the flavone may occur in aqueous solution) or in the solid state and to increase its bio-availability, we first envisaged to stabilize the flavone by metal complexation with a biological relevant redox-inert metal cation such as magnesium(II) or calcium(II), which are used in therapeutic formulations in human medicine. Mg(II) coordination properties with BJ486K (newly re-prepared as XM148) using MgCl₂ was first examined. As the last step of the improved synthetic route to BJ486K involves deprotection of the 5-methoxyl group with the Lewis acid MgBr₂, we anticipated that the deprotection/complexation steps may be performed in one pot, thus constituting a smart formulation approach. Because of the poor solubility of the magnesium salt in CH₃CN (solvent used for the deprotection reaction), we used, for the physico-chemical approach, a mixed solvent made of 50% of CH₃CN and 50 % of water to ensure solubilization of both reactants (XM148 and MgCl₂). Figure 17A depicted the spectral variations of the flavone-centered absorptions induced by Mg(II) chelation. Statistical analysis of the spectral data allowed us to calculate the stability constant of the XM148-MgCl₂ complex (log *K* = 2.4 ± 0.3) as well as its electronic spectral properties (Figure 17B). The flavone XM148 (= BJ486K) is characterized by two absorption maxima lying at 283 nm (band II) and 333 nm (Band I) assigned to the π-π* transitions of the B and A aromatic systems, respectively. Coordination of Mg(II) by the β-hydroxy-ketone bidentate site of BJ486K induced a bathochromic shift of the band II which apparently tends to blend with band I as well as formation of the new band in the visible region of much lower intensity. The spectroscopic signatures are unambiguous features of BJ486K (XM148) metal coordination processes.

As an additional evidence of Mg(II) complex formation through the β-hydroxy-ketone bidentate site of BJ486K, we conducted an absorption spectrophotometric titration of the final precursor **B** (= XM131 - the 5-OH is protected with a methyl group) by MgCl₂ under comparable experimental conditions than those employed for BJ486K (Figure 18). As anticipated, no Mg(II) complex is observed even under a large excess of Mg(II) thus further demonstrating that the β-hydroxy-ketone bidentate site of BJ486K is the metal chelation site.

The knowledge of the thermodynamic parameters of BJ486K/MgCl₂ then allowed us to simulate the experimental conditions of a potential one-pot reaction (deprotection/complexation) of XM131 (the final precursor of BJ486K) with MgCl₂ (Figure 19). The relatively low stability of the BJ486K-MgCl₂ complex indicated that a significant excess of MgCl₂ is indeed required for formation of more than 95% of magnesium(II) complex (Figure 19) for [BJ486K] = 0.016 M (our standard experimental conditions for low scale preparation). Even though addition of excess of MgCl₂ will undoubtedly accelerate the rate of the deprotection reaction (MgCl₂ is indeed much less reactive than MgBr₂), the final formulation will consequently contain high excess of MgCl₂ and the low coordination stability might hamper flavone/MgCl₂ accurate content determination.

To bypass these thermodynamic drawbacks, we then decided to discriminate the protection from the formulation (metal complex formation) steps. The final precursor XM131 was first reacted with MgBr₂ to afford the pure BJ486K flavone (see improved synthetic route to BJ486K). BJ486K was then reacted in a last step with a biocompatible magnesium salt. Among the numerous magnesium(II) salts safely employed in pharmacological compositions (Mg(II) stearate, Mg(II) pidolate, Mg(II) lactate...), we first focused our attention on Mg(II) stearate as a proof of concept. Magnesium stearate melts at about 120 °C, is not soluble in water, and is generally considered safe for human consumption at levels below 2500 mg/kg per day. Magnesium stearate is also often used as an excipient in the manufacture of medical tablets, capsules and powders and might significantly decrease the release time of the active ingredients in tablets, which would significantly increase the rather low bio-availability of BJ486K (t_{1/2} ∼ 7 min in mice). To evaluate the stability of the Mg(II)-BJ486K complex when stearic acids are used as auxiliary ligands, we carried out an absorption spectrophotometric titration of BJ486K in pure dioxane. To circumvent the low solubility of Mg(II) stearate in dioxane, a concentrated stock solution was refluxed in this solvent and aliquots were added to a BJ486K solution in a quartz optical cell. Formation of the Mg(II) complex was monitor using absorption spectrophotometry. Figure 20 displays the absorption spectrophotometric data. Even though the experimental conditions are different, the stability of the BJ486K-Mg(stearate)₂ complex (log *K* = 4.1 ± 0.6) is two orders of magnitude higher with respect to that of BJ486K-MgCl₂ (log *K* = 2.4 ± 0.3). This stabilization is clearly the result of the auxiliary stearate ligands and supports well our first hypotheses. The distribution diagrams provided in Figure 20 indicate that under the same conditions of reaction ([BJ486K] = 0.016 M), addition of only one equivalent would lead to the formation of more than 93% of the targeted complex, which can be purified and analyzed more conveniently. The experimental synthetic section describing the synthesis and characterization of the magnesium BJ486K complex under its stearate salt is given in Figures 20A and 20B.

Distribution diagrams calculated for the ternary Mg(stearate)₂ : BJ486K complex allowed the evaluation of the amount of the magnesium(II) complex (93%) in solution (Figure 21).

To protect the flavone from oxidative degradation, the preparation of a magnesium complex was investigated. To form the complex, 1 equivalent of BJ486K was dissolved in 1,4-dioxane to give a yellow solution. Then 0.95 equivalents of magnesium stearate are added. The mixture was refluxed for 1 h. The final solution became orange, likely because of the formation of the complex. This observation correlated well with the results obtained from the UV-Vis physico-chemical study (apparition of a low intensity band in the visible region). The obtained yellow-orange solid was found to be insoluble in most of the organic solvents commonly used. Efforts are now directed towards full characterization of the magnesium(II) stearate/BJ486K complex. First evidence that the Mg(II) stearate/flavone formulation is successful came from Infra Red spectroscopy measurements. IR spectra of the Mg(II) stearate, the flavone and the Mg(II) stearate/BJ486K complex were recorded and are provided in Figure 22A.
It can be observed that the hydroxy stretching band appearing at 3157 cm⁻¹ for the free flavone (5-OH) disappears completely once the flavone is complexed to the magnesium(II) (Figure 22B). This is a clear proof that a chelation has taken place (Jose, C.I. et al., Spectrochimica Acta 1974, 30A, 1199) . Also, further proof of complexation can be stated by comparing the bands corresponding to the C=O stretching. Indeed, 5-hydroxy-flavonato complexes show v(CO) bands shifting towards a lower frequency by 14-35 cm⁻¹, indicating that the 5-hydroxy-flavone is coordinated to the metal ion as a bidentate chelate (Figure 22C) (Hiraki, K. et al., Bull. Chem. Soc. Jap. 1978, 51, 2425). Indeed, it can be observed that from Figure 22C for the first C=O band lying at 1675 cm⁻¹, there is a Δν(CO) of about 22 cm⁻¹ and for the second band centered at 1608 cm⁻¹, there is a Δν(CO) of about 16 cm⁻¹, which correlates perfectly with a complexed flavone. Lastly, it is clear that no free stearic acid is present in the sample, because it has a very characteristic v(CO) band at about 1700 cm⁻¹, which does not appear in the IR spectrum of the complexed flavone. By contrast, the IR signature of the complexed stearate are still present for the Mg(II)/BJ486K complex indicating that these long chain alkyl-carboxylate binders are still involved in the Mg(II) coordination.

### Choice of a solvent or of a vehicle to test the final metal complexes in cell assays and in murine models, in particular the the XM148-Mg-Stearate complex (XM150)

Cremophor^{EL} (from BASF) in mixture with DMSO was used to solubilize the **XM148**-Mg-Stearate complex (**XM150**). The protocol for solubilizing is as followed:
Prepare 10 mM solution of **XM150** in pure cremophor^{EL}, then dilute to ½ with pure DMSO and let the sample at 60 °C in a rotative incubator (slow stirring) overnight. The day of the experiment, aqueous saline phosphate buffer (PBS) maintained at 60 °C was added, and the homogeneous solution was slowly diluted in a rotative incubator to final 1 mM of **XM150** in aqueous PBS.

## Claims

1. A compound of the general formula (I): 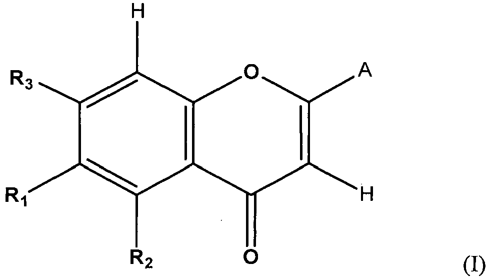 or a pharmacologically acceptable salt, solvate, or hydrate thereof, wherein
A is: 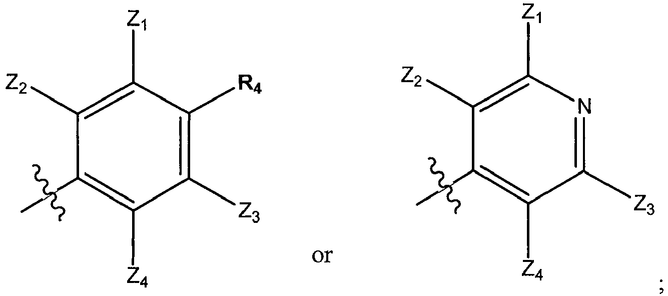 R₁ is a group OR₅ and R₂ is an oxygen atom that is taken together with the β-carbonyl unit to form a metal complex of the general formula (II): 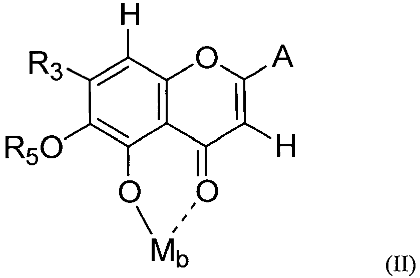 wherein
M_{b} is a non electroactive metal ion under its divalent state selected from zinc, magnesium, calcium, copper, or M is trivalent iron; and
R₅ is a hydrogen atom or an acetyl group;
R₃ is a C₁-C₆ alkyl or a C₁-C₆ alkoxy group;
R₄ is a nitro, cyano, sulphur pentafluoride, -SO₃H, -SO₂NH₂, an optionally substituted C₁-C₆ alkyl or an optionally substituted C₁-C₆ alkoxy group, which may be substituted with one or more fluorine, chlorine, bromine or iodine atoms or by -OH, =O, -SH, =S, NH₂, =NH or -NO₂ group(s); and
Z₁, Z₂, Z₃, and Z₄, each and independently of each other represent a hydrogen atom, halogen atom, cyano, nitro, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, or a compound selected from:

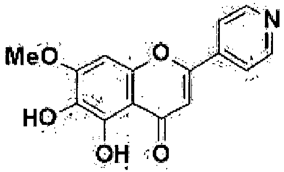

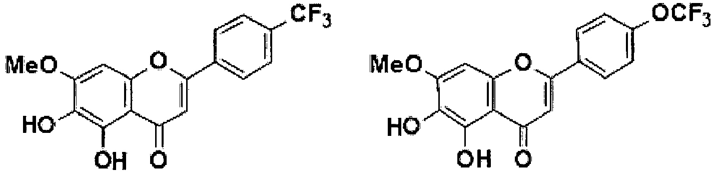

2. The compound according to claim 1, wherein A is 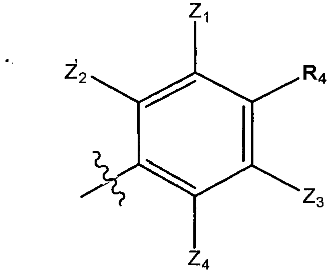 or a pharmacologically acceptable salt, solvate, or hydrate thereof.

3. The compound according to claim 1, wherein A is: 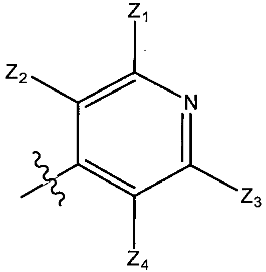 or a pharmacologically acceptable salt, solvate, or hydrate thereof.

4. The compound according to any one of claims 1 or 2, wherein R₄ is OMe, CF₃, OCF₃, fluorine, chlorine, bromine, or -SO₃H, or a pharmacologically acceptable salt, solvate, or hydrate thereof.

5. The compound according to any one of claims 1 to 4, wherein R₃ is methyl or OMe, or a pharmacologically acceptable salt, solvate, or hydrate thereof.

6. The compound according to any one of claims 1 to 5, wherein Z₁ to Z₄ are each and independently selected from hydrogen atom, fluorine, chlorine, bromine, and C₃-C₆ alkyl, or a pharmacologically acceptable salt, solvate, or hydrate thereof.

7. Compound according to any one of claims 1 to 6, selected from: 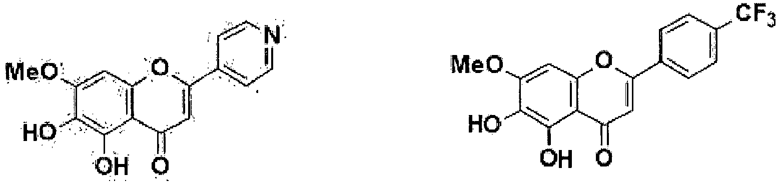 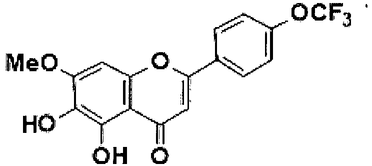 a compound of formula: 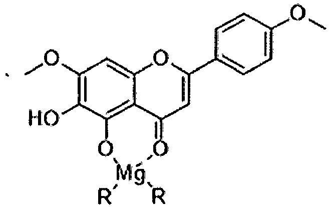 wherein R is stearate or Cl, or is selected from the 5,6-Dihydroxy-7-methoxy-2-(4-methoxy-phenyI)-chromen-4-one (BJ486K):Fe(III) complex and the 5,6-Dihydroxy-7-methoxy-2-(4-methoxy-phenyI)-chromen-4-one:Fe(III):NTA complex.

8. Pharmaceutical composition comprising at least one compound according to any one of claims 1 to 7, and, optionally, at least one pharmaceutically acceptable carrier(s) and/or at least one customary excipient.

9. Pharmaceutical composition according to claim 8, comprising at least one compound selected from: 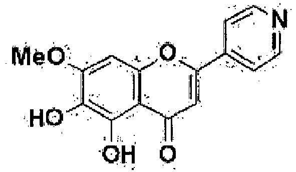 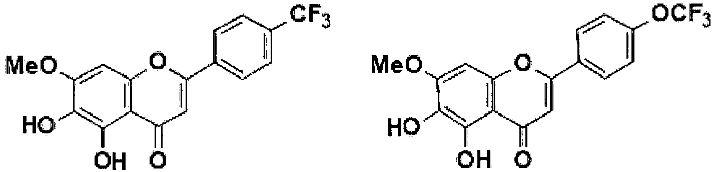 or one compound of formula: 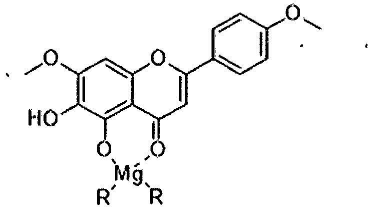 wherein R is stearate or Cl,
or one compound selected from the 5,6-Dihydroxy-7-methoxy-2-(4-methoxy-phenyI)-chromen-4-one:Fe(III) complex and the 5,6-Dihydroxy-7-methoxy-2-(4-methoxy-phenyI)-chromen-4-one:Fe(III):NTA complex.

10. Compound according to any one of claims 1 to 7, or the compound: 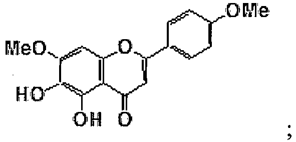 or a pharmaceutical composition containing one or more of said compounds for use in the prevention and/or treatment of a viral infection.

11. Compound for use in the prevention and/or treatment of a viral infection according to claim 10, wherein the compound is selected from: 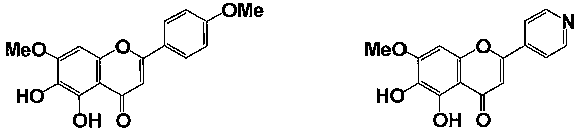 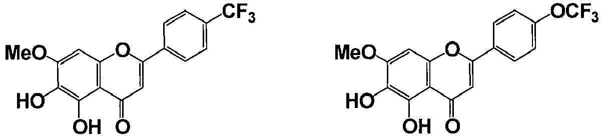 or is a compound of formula: 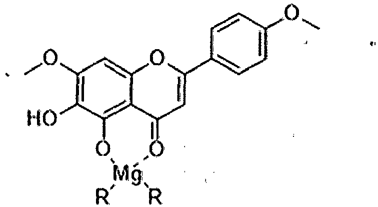 wherein R is stearate or Cl,
or is selected from the 5,6-Dihydroxy-7-methoxy-2-(4-methoxy-phenyI)-chromen-4-one:Fe(III) complex and the 5,6-Dihydroxy-7-methoxy-2-(4-methoxy-phenyI)-chromen-4-one:Fe(III):NTA complex.

12. The compound for use in the prevention and/or treatment of a viral infection according to claim 10 or 11, wherein the compound is

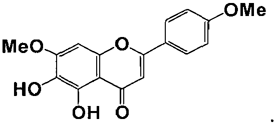

13. The compound for use in the prevention and/or treatment of a viral infection according to any of claims 10 to 12, wherein the infection is an infection by an enveloped virus.

14. A combination preparation for use in the treatment or prophylaxis of a viral infection containing at least one compound according to any of claims 1 to 7, or the compound: 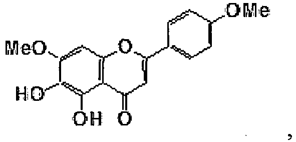 or a pharmacologically acceptable salt, solvate, or hydrate thereof, and at least one further active pharmaceutical ingredient.

15. A combination preparation for use in the treatment or prophylaxis of a viral infection according to claim 14 containing at least one compound selected from: 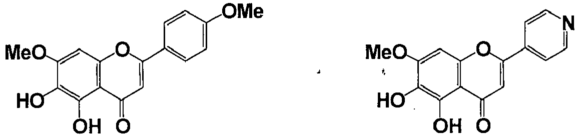 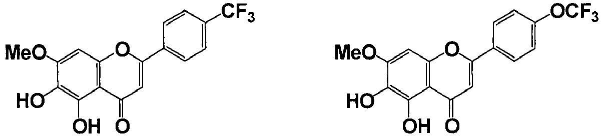 or one compound of formula: 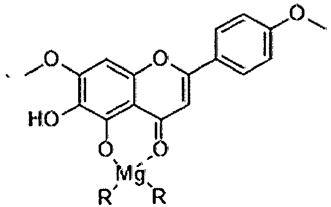 wherein R is stearate or Cl,
or one compound selected from the 5,6-Dihydroxy-7-methoxy-2-(4-methoxy-phenyl)-chromen-4-one:Fe(III) complex and the 5,6-Dihydroxy-7-methoxy-2-(4-methoxy-phenyI)-chromen-4-one:Fe(III):NTA complex.

16. The combination preparation of Claim 14 or 15, wherein the further active pharmaceutical ingredient is selected from cyclosporine A, pegylated interferon-alpha-2a, pegylated interferon-alpha-2b, ribavirin, viramidine, boceprevir (victrelis), telaprevir (Incivo), SP 30, ITX 5061, RG7128, PSI-7977, NM 283, Albuferon and/or Zadaxin.

## Patentansprüche

1. Eine Verbindung der allgemeinen Formel (I): 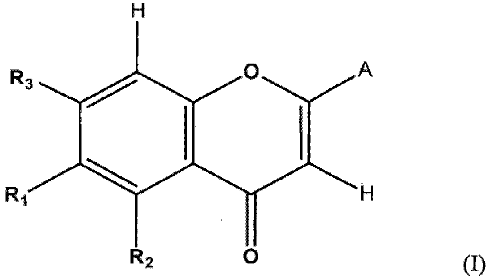 oder ein pharmakologisch-verträgliches Salz, Solvat oder Hydrat davon, wobei A: 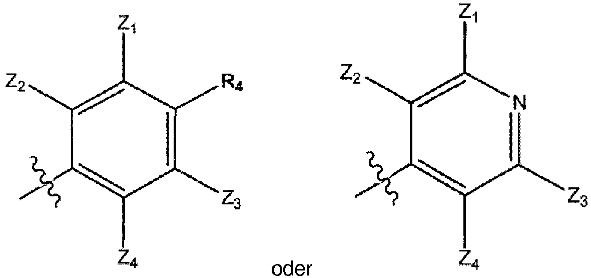 ist;
R₁ ein Rest OR₅ ist und R₂ ein Sauerstoffatom ist, das zusammen mit der β-Carbonyl-Einheit genommen wird, um einen Metallkomplex der allgemeinen Formel (II) zu bilden: 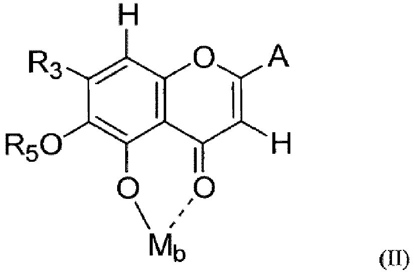 wobei
M_{b} ein nicht elektroaktives Metallion in seinem zweiwertigen Zustand ausgewählt aus Zink, Magnesium, Calcium, Kupfer ist,
oder M dreiwertiges Eisen ist; und
R₅ ein Wasserstoffatom oder ein Acetylrest ist;
R₃ eine C₁-C₆-Alkyl- oder ein C₁-C₆ Alkoxyrest ist;
R₄ ein Nitro, Cyano, Schwefelpentafluorid, -SO₃H, -SO₂NH₂, ein gegebenenfalls substituierter C₁-C₆ Alkyl- oder ein gegebenenfalls substituierter C₁-C₆ Alkoxyrest ist, welcher mit einem oder mehreren Fluor-, Chlor-, Brom-, oder lod-Atomen oder durch -OH, =O, -SH, =S, NH₂, =NH oder -NO₂-Rest(e) substituiert sein kann; und
Z₁, Z₂, Z₃ und Z₄ jeweils und unabhängig voneinander ein Wasserstoffatom, Halogenatom, Cyano-, Nitro-, einen Alkyl-, einen Alkenyl-, einen Alkynyl-, einen Heteroalkyl-, einen Cycloalkyl-, einen Heterocycloalkyl-, einen Alkylcycloalkyl-, einen Heteroalkylcycloalkyl-, einen Aryl-, einen Heteroaryl-, einen Aralkyl- oder einen Heteroaralkylrest darstellen,
oder eine Verbindung ausgewählt aus:

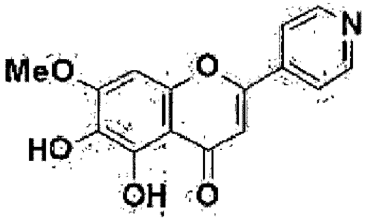

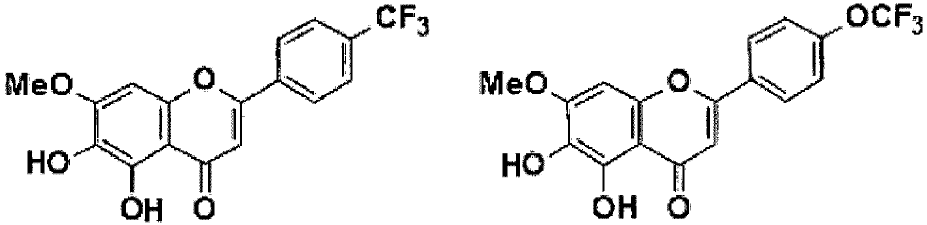

2. Die Verbindung gemäß Anspruch 1, wobei A 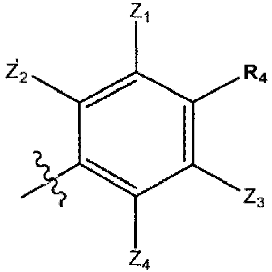 oder ein pharmakologisch-verträgliches Salz, Solvat oder Hydrat davon ist.

3. Die Verbindung gemäß Anspruch 1, wobei A: 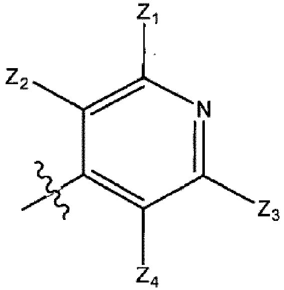 oder ein pharmakologisch-verträgliches Salz, Solvat oder Hydrat davon ist.

4. Die Verbindung gemäß einem der Ansprüche 1 oder 2, wobei R₄ gleich OMe, CF₃, OCF₃, Fluor, Chlor, Brom oder -SO₃H, oder ein pharmakologisch-verträgliches Salz, Solvat oder Hydrat davon ist.

5. Die Verbindung gemäß einem der Ansprüche 1 bis 4, wobei R₃ gleich Methyl oder OMe oder ein pharmakologisch-verträgliches Salz, Solvat oder Hydrat davon ist.

6. Die Verbindung gemäß einem der Ansprüche 1 bis 5, wobei Z₁ bis Z₄ jeweils und unabhängig ausgewählt sind aus Wasserstoffatom, Fluor, Chlor, Brom und C₃-C₆-Alkyl oder einem pharmakologisch-verträglichen Salz, Solvat oder Hydrat davon.

7. Verbindung gemäß einem der Ansprüche 1 bis 6, ausgewählt aus: 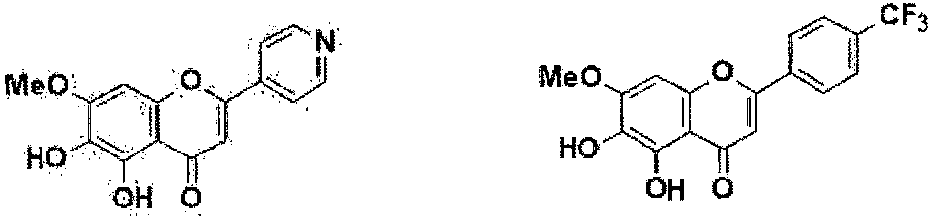 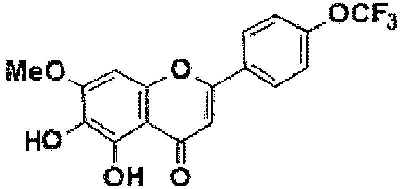 einer Verbindung der Formel: 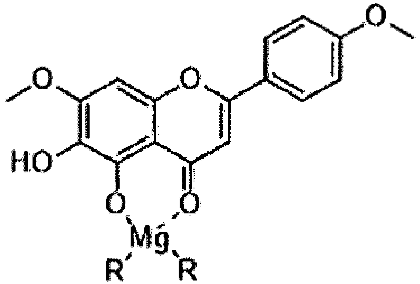 wobei R gleich Stearat oder Cl ist oder ausgewählt ist aus
dem 5,6-Dihydroxy-7-methoxy-2-(4-methoxy-phenyl)-chromen-4-one (BJ486K):Fe(III)-Komplex und dem 5,6-Dihydroxy-7-methoxy-2-(4-methoxy-phenyl)-chromen-4-one:Fe(III):NTA-Komplex.

8. Pharmazeutische Zusammensetzung, umfassend mindestens eine Verbindung gemäß einem der Ansprüche 1 bis 7 und gegebenenfalls mindestens einen pharmazeutisch-verträglichen Träger und/oder mindestens einen üblichen Hilfsstoff.

9. Pharmazeutische Zusammensetzung gemäß Anspruch 8, umfassend mindestens eine Verbindung ausgewählt aus: 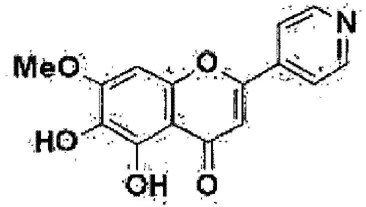 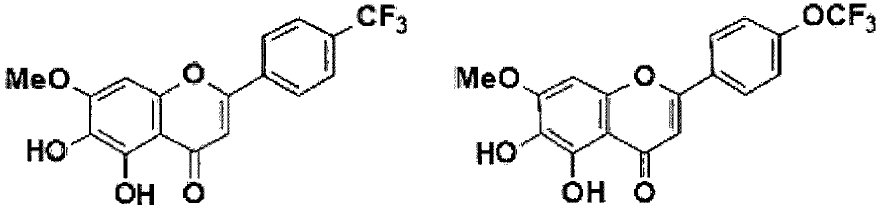 oder einer Verbindung der Formel: 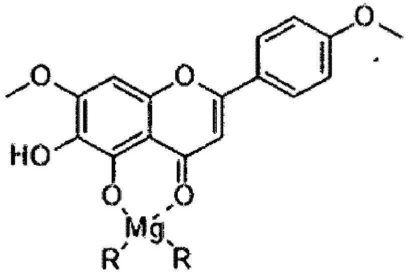 wobei R gleich Stearat oder Cl ist
oder eine Verbindung ausgewählt aus dem 5,6-Dihydroxy-7-methoxy-2-(4-methoxy-phenyl)-chromen-4-one:Fe(III)-Komplex und dem 5,6-Dihydroxy-7-methoxy-2-(4-methoxy-phenyl)-chromen-4-one:Fe(III):NTA-Komplex.

10. Verbindung gemäß einem der Ansprüche 1 bis 7, oder die Verbindung: 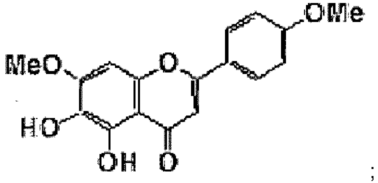 oder eine pharmazeutische Zusammensetzung, enthaltend ein oder mehrere der Verbindungen zur Verwendung in der Prävention und/oder Behandlung einer viralen Infektion.

11. Verbindung zur Verwendung in der Prävention und/oder Behandlung einer viralen Infektion gemäß Anspruch 10, wobei die Verbindung ausgewählt ist aus: 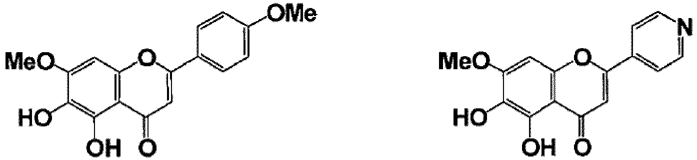 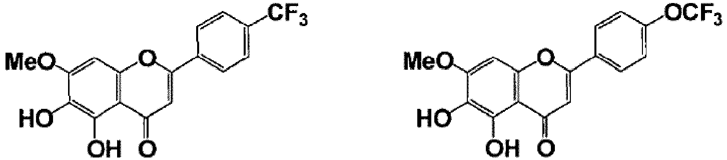 oder eine Verbindung der Formel: 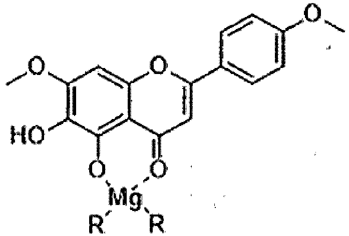 ist,
wobei R gleich Stearat oder Cl ist,
oder ausgewählt ist aus dem 5,6-Dihydroxy-7-methoxy-2-(4-methoxy-phenyl)-chromen-4-one:Fe(III)-Komplex und dem 5,6-Dihydroxy-7-methoxy-2-(4-methoxy-phenyl)-chromen-4-one:Fe(III):NTA-Komplex.

12. Die Verbindung zur Verwendung in der Prävention und/oder Behandlung einer viralen Infektion gemäß Anspruch 10 oder 11, wobei die Verbindung 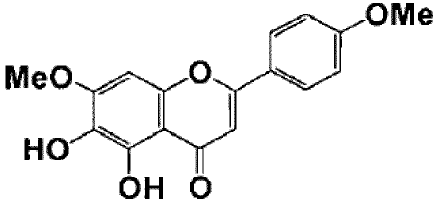 ist.

13. Die Verbindung zur Verwendung in der Prävention und/oder Behandlung einer viralen Infektion gemäß einem der Ansprüche 10 bis 12, wobei die Infektion eine Infektion durch einen umhüllten Virus ist.

14. Ein Kombinationspräparat zur Verwendung in der Behandlung oder Prophylaxe einer viralen Infektion, enthaltend mindestens eine Verbindung gemäß einem der Ansprüche 1 bis 7 oder die Verbindung: 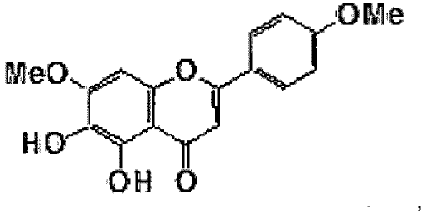 oder ein pharmakologisch-verträgliches Salz, Solvat oder Hydrat davon und mindestens einen weiteren aktiven pharmazeutischen Bestandteil.

15. Ein Kombinationspräparat zur Verwendung in der Behandlung oder Prophylaxe einer viralen Infektion gemäß Anspruch 14, enthaltend mindestens eine Verbindung ausgewählt aus: 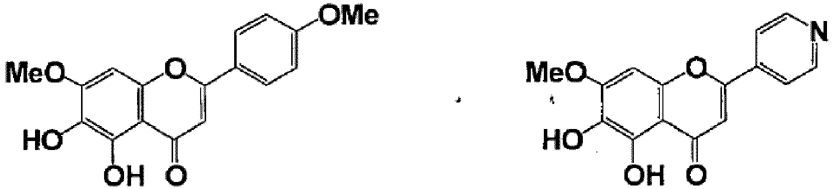 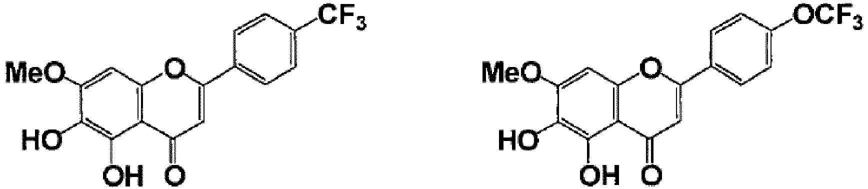 oder eine Verbindung der Formel: 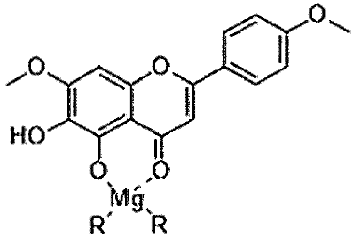 wobei R gleich Stearat oder Cl ist,
oder eine Verbindung ausgewählt aus dem 5,6-Dihydroxy-7-methoxy-2-(4-methoxy-phenyl)-chromen-4-one:Fe(III)-Komplex und dem 5,6-Dihydroxy-7-methoxy-2-(4-methoxy-phenyl)-chromen-4-one:Fe(III):NTA-Komplex.

16. Das Kombinationspräparat gemäß Anspruch 14 oder 15, wobei der weitere aktive pharmazeutische Bestandteil ausgewählt ist aus Cyclosporin A, pegyliertem Interferon-alpha-2a, pegyliertem Interferon-alpha-2b, Ribavirin, Viramidin, Boceprevir (Victrelis), Telaprevir (Incivo), SP 30, ITX 5061, RG7128, PSI-7977, NM 283, Albuferon und/oder Zadaxin.

## Revendications

1. Composé de formule générale (I) : 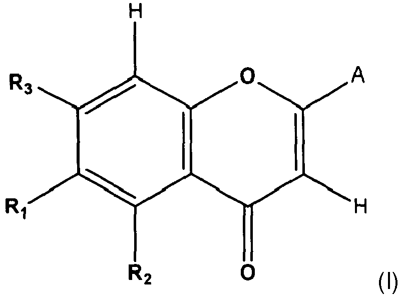 ou un sel, solvate ou hydrate de celui-ci acceptable du point de vue pharmacologique, formule dans laquelle
A est : 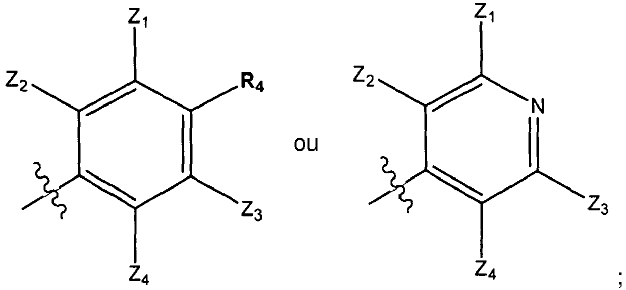 R₁ est un groupe OR₅ et R₂ est un atome d'oxygène qui est pris conjointement avec le motif β-carbony|e pour former un complexe métallique de formule générale (II): 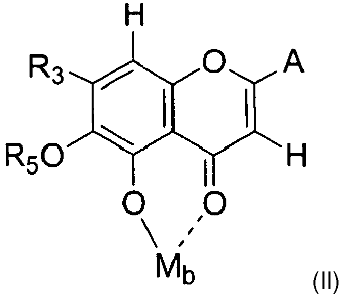 dans laquelle
M_{b} est un ion métallique non électroactif dans son état divalent, choisi parmi le zinc, le magnésium, le calcium, le cuivre, ou bien M est le fer trivalent ; et
R₅ est un atome d'hydrogène ou un groupe acétyle ;
R₃ est un groupe alkyle en C₁ à C₆ ou alcoxy en C₁ à C₆ ;
R₄ est un groupe nitro, cyano, pentafluorure de soufre, -SO₃H, -SO₂NH₂, alkyle en C₁ à C₆ éventuellement substitué ou alcoxy en C₁ à C₆ éventuellement substitué, qui peut être substitué par un ou plusieurs atomes de fluor, chlore, brome ou iode ou par un ou plusieurs groupes -OH, =O, -SH, =S, NH₂, =NH ou -NO₂ ; et
chacun de Z₁, Z₂, Z₃ et Z₄, indépendamment des autres, représente un atome d'hydrogène, un atome d'halogène, un groupe cyano, nitro, alkyle, alcényle, alcynyle, hétéroalkyle, cycloalkyle, hétérocycloalkyle, alkylcycloalkyle, hétéroalkylcycloalkyle, aryle, hétéroaryle, aralkyle ou hétéroaralkyle, ou un composé choisi parmi :

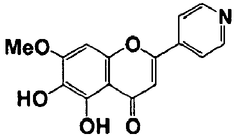

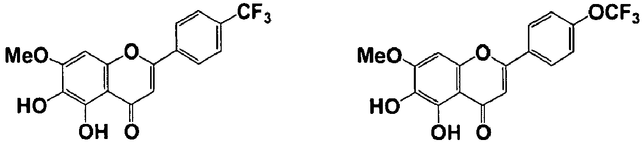

2. Composé selon la revendication 1, dans lequel A est 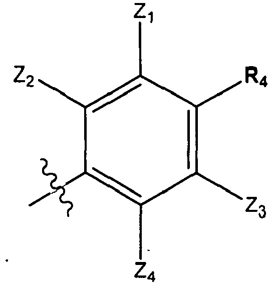 ou un sel, solvate ou hydrate de celui-ci acceptable du point de vue pharmacologique.

3. Composé selon la revendication 1, dans lequel A est : 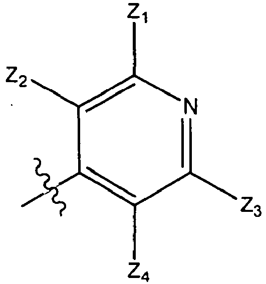 ou un sel, solvate ou hydrate de celui-ci acceptable du point de vue pharmacologique.

4. Composé selon l'une quelconque des revendications 1 et 2, dans lequel R₄ est OMe, CF₃, OCF₃, le fluor, le chlore, le brome ou -SO₃H, ou un sel, solvate ou hydrate de celui-ci acceptable du point de vue pharmacologique.

5. Composé selon l'une quelconque des revendications 1 à 4, dans lequel R₃ est méthyle ou OMe, ou un sel, solvate ou hydrate de celui-ci acceptable du point de vue pharmacologique.

6. Composé selon l'une quelconque des revendications 1 à 5, dans lequel chacun de Z₁ à Z₄ est indépendamment choisi parmi un atome d'hydrogène, le fluor, le chlore, le brome, et un alkyle en C₃ à C₆, ou un sel, solvate ou hydrate de celui-ci acceptable du point de vue pharmacologique.

7. Composé selon l'une quelconque des revendications 1 à 6, choisi parmi : 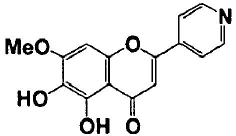 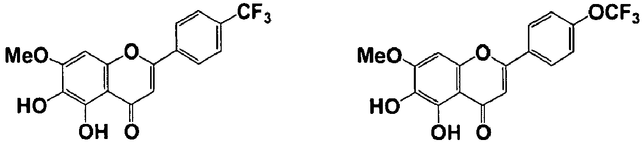 un composé de formule : 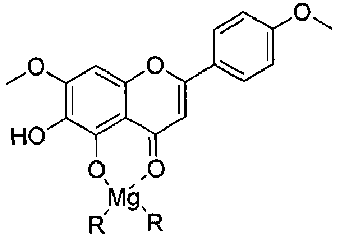 dans laquelle R est un stéarate ou Cl, ou est choisi parmi :
le complexe de 5,6-dihydroxy-7-méthoxy-2-(4-méthoxyphényl)chromén-4-one (BJ486K) et de Fe(III), et
le complexe de 5,6-dihydroxy-7-méthoxy-2-(4-méthoxyphényl)chromén-4-one, de Fe(III) et de NTA.

8. Composition pharmaceutique comprenant au moins un composé selon l'une quelconque des revendications 1 à 7 et éventuellement au moins un véhicule acceptable du point de vue pharmaceutique et/ou au moins un excipient habituel.

9. Composition pharmaceutique selon la revendication 8, comprenant au moins un composé choisi parmi : 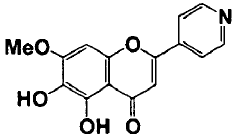 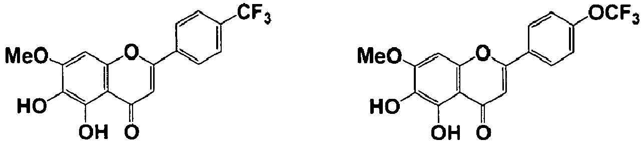 ou un composé de formule : 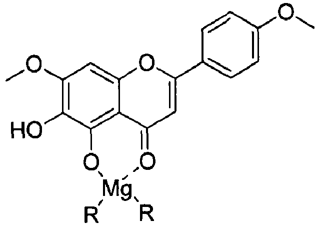 dans laquelle R est un stéarate ou Cl,
ou un composé choisi parmi le complexe de 5,6-dihydroxy-7-méthoxy-2-(4-méthoxyphényl)chromén-4-one et de Fe(III), et le complexe de 5,6-dihydroxy-7-méthoxy-2-(4-méthoxyphényl)chromén-4-one, de Fe(III) et de NTA.

10. Composé selon l'une quelconque des revendications 1 à 7, ou le composé : 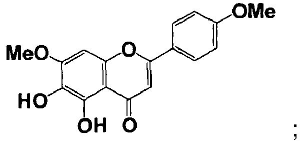 ou une composition pharmaceutique contenant un ou plusieurs desdits composés, pour une utilisation dans la prévention et/ou le traitement d'une infection virale.

11. Composé pour une utilisation dans la prévention et/ou le traitement d'une infection virale selon la revendication 10, lequel composé est choisi parmi : 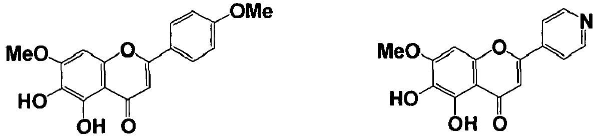 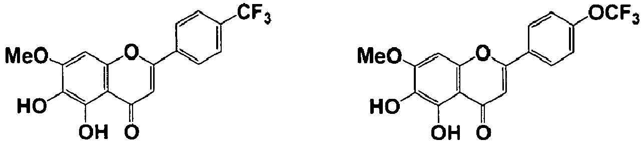 ou est un composé de formule : 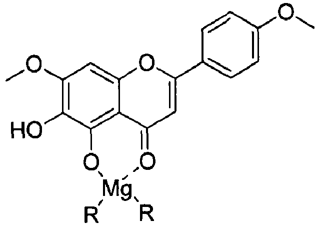 dans laquelle R est un stéarate ou Cl,
ou est choisi parmi le complexe de 5,6-dihydroxy-7-méthoxy-2-(4-méthoxyphényl)chromén-4-one et de Fe(III), et le complexe de 5,6-dihydroxy-7-méthoxy-2-(4-méthoxyphényl)chromén-4-one, de Fe(III) et de NTA.

12. Composé pour une utilisation dans la prévention et/ou le traitement d'une infection virale selon la revendication 10 ou 11, lequel composé est

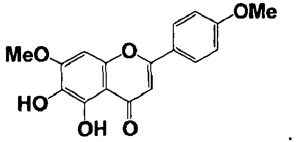

13. Composé pour une utilisation dans la prévention et/ou le traitement d'une infection virale selon l'une quelconque des revendications 10 à 12, dans lequel l'infection est une infection par un virus à enveloppe.

14. Préparation combinée pour une utilisation dans le traitement ou la prophylaxie d'une infection virale contenant au moins un composé selon l'une quelconque des revendications 1 à 7, ou le composé : 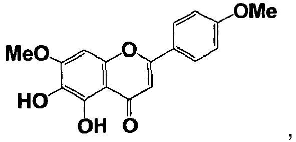 ou un sel, solvate ou hydrate de celui-ci acceptable du point de vue pharmacologique, et au moins un autre ingrédient pharmaceutique actif.

15. Préparation combinée pour une utilisation dans le traitement ou la prophylaxie d'une infection virale selon la revendication 14, contenant au moins un composé choisi parmi : 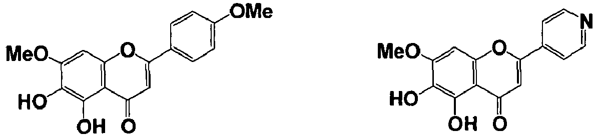 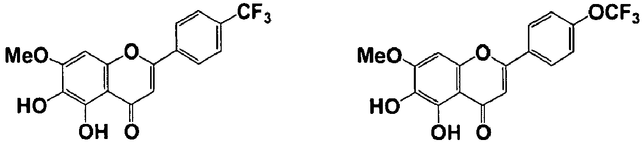 ou un composé de formule : 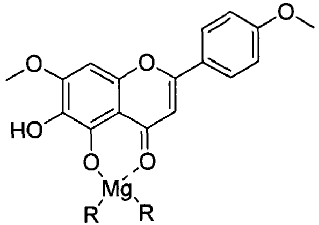 dans laquelle R est un stéarate ou Cl,
ou un composé choisi parmi le complexe de 5,6-dihydroxy-7-méthoxy-2-(4-méthoxyphényl)chromén-4-one et de Fe(III), et le complexe de 5,6-dihydroxy-7-méthoxy-2-(4-méthoxyphényl)chromén-4-one, de Fe(III) et de NTA.

16. Préparation combinée selon la revendication 14 ou 15, dans laquelle l'autre ingrédient pharmaceutique actif est choisi parmi la cyclosporine A, l'interféron-alpha-2a pégylé, l'interféron-alpha-2b pégylé, la ribavirine, la viramidine, le bocéprévir (victrelis), le télaprévir (Incivo), SP 30, ITX 5061, RG7128, PSI-7977, NM 283, l'albuféron et/ou la zadaxine.
